# EUROPEAN PATENT APPLICATION

(11) **EP 4 062 939 A1**
(43) Date of publication of application: **28.09.2022**
(21) Application number: 22172571.6
(22) Date of filing: 26.03.2014
(51) Int. Cl.: A61K 47/02, A61K 31/4045, A61K 31/56, A61P 11/00, A61P 25/06, A61M 15/08

(54) **NASAL ADMINISTRATION**

(30) Priority: 26.03.2013 US 201361805400 P
(62) Divisional of application: 14721008.2
(71) Applicant: Optinose AS, 0702 Oslo (NO)
(72) Inventor: DJUPESLAND, Per Gisle, 0772 Oslo (NO); MAHMOUD, Ramy A., Skillman, 08558 (US); MESSINA, John, Downingtown, 19335 (US)
(74) Representative: Boden, Keith McMurray

(57) **Abstract**

A therapeutic agent including fluticasone for treating chronic rhinosinusitis with or without nasal polyps in a patient and carbon dioxide as a pH reducing material in combination, characterized in that the fluticasone is delivered in amount of 100 µg to 400 µg twice daily and administration is by a delivery device comprising a mouthpiece and a nosepiece, by placing the mouthpiece into a mouth of the patient and the nosepiece into a nostril of the patient and the patient exhaling a breath into the mouthpiece to create a fluid flow out of the nosepiece into the nostril and deliver the fluticasone and the carbon dioxide from the nosepiece to a location within a nasal passage of the patient, wherein a pH in the nasal passage location is reduced by an amount ranging from about 0.1 pH units to about 0.2 pH units by controlling the fluid flow to deliver a concentration of the carbon dioxide of from about 5% vol/vol to about 6% vol/vol carbon dioxide at a fluid flow rate of at least 20 L/min for a duration of from about 2 seconds to about 3 seconds and the carbon dioxide is delivered before or at the same time as the fluticasone.

## Description

### Field of the Disclosure

The present disclosure, in one embodiment, relates to the nasal administration of substances, in particular drugs, and in particular substances which require a rapid onset of action, such as in the treatment of pain, including headaches, for example, cluster headaches and migraine, and neuropathic pain. It relates, in another embodiment, to nasal delivery of carbon dioxide gas or nasal pH adjustment as a supplement a therapeutic treatment, such as for the treatment of pain.

### SUMMARY OF THE DISCLOSURE

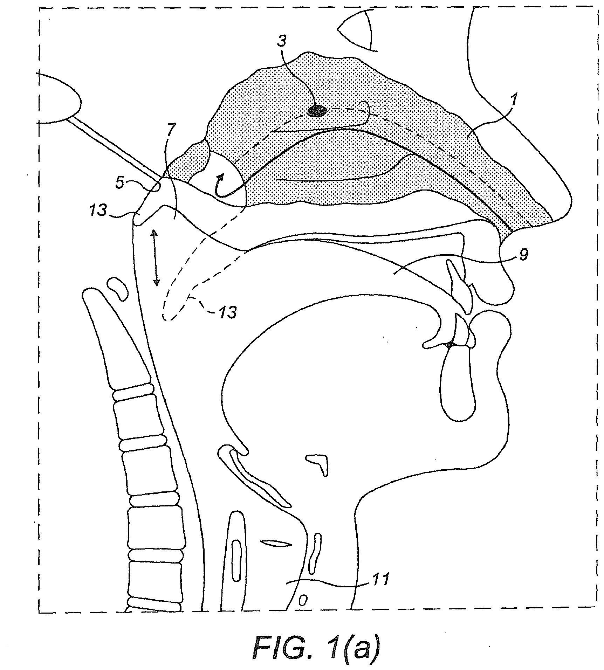

Referring to Figure 1(a), the nasal airway 1 comprises the two nasal cavities separated by the nasal septum, which airway 1 includes numerous ostia, such as the paranasal sinus ostia 3 and the tubal ostia 5, and olfactory cells, and is lined by the nasal mucosa. The nasal airway 1 can communicate with the nasopharynx 7, the oral cavity 9 and the lower airway 11, with the nasal airway 1 being in selective communication with the anterior region of the nasopharynx 7 and the oral cavity 9 by opening and closing of the oropharyngeal velum 13. The velum 13, which is often referred to as the soft palate, is illustrated in solid line in the closed position, as achieved by providing a certain positive pressure in the oral cavity 9, such as achieved on exhalation through the oral cavity 9, and in dashed line in the open position.

The present inventors have surprisingly identified that a rapid systemic uptake and a rapid response rate can be achieved, as compared, for example, to the conventional delivery of an equivalent liquid substance, by the delivery of substance and at least one gas to the posterior region of the nasal airway.

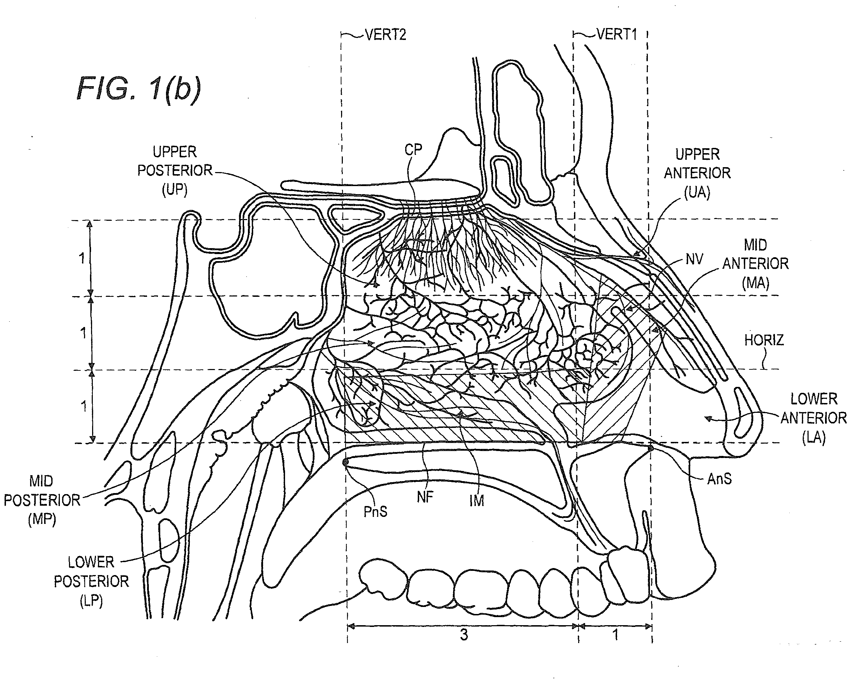

The posterior region of the nasal airway is that region which is posterior of the nasal valve NV, as illustrated in Figure 1(b). The nasal valve comprises the anterior bony cavum which contains inferior turbinate erectile tissue and septal erectile tissue, which are supported respectively by compliant ala tissue and the rigid cartilaginous septum (Cole, P (The Respiratory Role of the Upper Airways, a selective clinical and pathophysiological review. 1993, Mosby-Year Book Inc. ISBN1.55664-390-X)). These elements combine to form a dynamic valve, which extends over several millimeters, that adjusts nasal airflow, and is stabilized by cartilage and bone, modulated by voluntary muscle and regulated by erectile tissue. The lumen of the nasal valve is the section of narrowest cross-sectional area between the posterior and anterior regions of the nasal airway, and is much longer and narrower dorsally than ventrally, and this lumen defines a triangular entrance which extends to the piriform region of the bony cavum. The nasal valve is lined in its anterior part with transitional epithelium, with a gradual transition posterior to respiratory epithelium. The nasal valve and anterior vestibule define roughly the anterior one-third of the nose.

The posterior region of the nasal airway is that region which is lined with respiratory epithelium, which is ciliated, and olfactory epithelium, which comprises nerves which extend downwards through the cribiform plate CP from the olfactory bulb, whereas the anterior region of the nasal airway is that region which is lined with squamous epithelium, which is not ciliated, and transitional epithelium. The olfactory epithelium extends on both the lateral and medial sides of the nasal airway, and typically extends downwards about 1.5 to 2.5 cm.

The upper posterior region is the region above the inferior meatus IM, as illustrated in Figure 1(b), and encompasses the middle turbinate, the sinus ostia in infundibulum (ostia to maxillary, frontal and ethmoidal sinuses), the olfactory region, and the upper branches of the trigeminal nerve, and is that region which includes veins which drain to the venous sinuses that surround the brain.

As illustrated in Figure 1(b), the posterior region of the nasal airway Is the nasal region posterior of an imaginary vertical plane VERT1 which is located at a position corresponding to one-quarter of the distance between the anterior nasal spine AnS, which is a pointed projection at the anterior extremity of the intermaxillary suture, and the posterior nasal spine PnS, which is the sharp posterior extremity of the nasal crest of the hard palate and represents the transition between the nose and the nasopharynx, which corresponds to a distance posterior of the anterior nasal spine AnS of between about 13 mm and about 14 mm (Rosenberger, H (Growth and Development of the Naso-Respiratory Area in Childhood, PhD Thesis, Laboratory of Anatomy, School of Medicine, Western Reserve University, Presented to the Annual Meeting of the American Laryngological, Rhinological and Otological Society, Charleston, South Carolina, USA, 1934) defines the distance between the anterior nasal spine AnS and the posterior nasal spine PnS as being 56 mm in eighteen year old boys and 53.3 mm in eighteen year old girls). As again illustrated in Figure 1(b), the posterior nasal region is bounded posteriorly by an imaginary vertical plane VERT2 which extends through the posterior nasal spine PnS.

As further illustrated in Figure 1(b), the upper region of the nasal airway is an upper segment of the nasal airway which is bounded by the cribiform plate CP and a horizontal plane HORIZ which is located at a position corresponding to one-third of the distance between the nasal floor NF of the nasal airway and the cribiform plate CP, which corresponds to a height of typically between about 13 and about 19 mm above the nasal floor NF (Zacharek, M A et al (Sagittal and Coronal Dimensions of the Ethmoid Roof: A Radioanatomic Study, Am J Rhino! 2005, Vol 19, pages 348 to 352) define the distance from the nasal floor NF to the cribiform plate CP as 46 +/- 4 mm). The upper posterior region can thus include an upper posterior region which may be bounded by the above-defined vertical and horizontal planes VERT1, HORIZ.

Gas therapy for the treatment of headaches, allergies, asthma and other conditions as well as associated physiologies is described in the following references in the literature, including Casale et al, J Allergy Clin Immunol 121 (1): 105-109 (2008), Vause et al, Headache 47: 1385-1397 (2007), Tzabazis et al, Life Science 87: 36-41 (2010), and Casale et al, Ann Allergy Asthma Immunol 107: 364-370 (2011).

WO-A-2001/064280 discloses methods and devices for transcutaneous and transmucosal applications of carbon dioxide in the form of gas and in the form of capnic solution (such as carbonated water) for the relief of pain, including musculoskeletal disorders, neuralgias, rhinitis and other ailments.

US-A-2011/0046546 discloses apparatus, methods and kits for treating symptoms associated with common ailments, such as headaches, rhinitis, asthma, epilepsy, nervous disorders and the like.

The present inventors have recognized that the administration of a combination of a therapeutic substance, and control of pH, pressure and/or NO concentration, such as by way of delivery of a gas through the nasal airway, can provide for an improved therapeutic treatment, in particular a surprisingly rapid onset of action of the therapeutic substance.

In one aspect the present disclosure provides a method of administering a substance to a subject, comprising the steps of delivering a substance to a posterior region of the nasal cavity of the subject, the posterior region comprising mucosa innervated by a trigeminal nerve; adjusting a pH of the mucosa, before, during or after the delivery of the substance, whereby a rate of uptake of the substance is increased.

In one embodiment the mucosa is further innervated by the sphenopalatine ganglion.

In one embodiment the substance is delivered through a nosepiece fitted to a nostril, optionally being a fluid-tight seal with a nare of the nostril.

In one embodiment the substance is delivered through a single nostril to the mucosa one trigeminal nerve.

In one embodiment the substance is delivered successively through each of the nostrils to the mucosa at each of the trigeminal nerves.

In one embodiment the pH is adjusted by delivery of at least one gas.

In one embodiment the at least one gas is delivered in a flow, optionally having a concentration of at least 5 vol% of the at least one gas.

In one embodiment the at least one gas comprises carbon dioxide.

In one embodiment adjustment of the pH mediates activity at the V1 branch of the trigeminal nerve.

in one embodiment the pH adjustment is performed during an event in which there is a parasympathaetic influence on the autonomic nervous system, by which the trigeminal nerve is predisposed to the pH adjustment and uptake of substance is increased.

In one embodiment the pH is reduced in the pH adjustment step.

In one embodiment the method further comprises the step of: adjusting a pressure in the nasal cavity before, during or after the delivery of the substance, whereby a rate of uptake of the substance is increased.

In one embodiment the pressure is at least about 3kPa, optionally from about 3 to about 7 kPa.

In one embodiment the pressure Is adjusted by delivery of at least one gas.

In one embodiment, the at least one gas is delivered in a flow, optionally having a concentration of at least 5 vol% of the at least one gas.

In one embodiment, the at least one gas comprises carbon dioxide.

In one embodiment the pressure adjustment mediates activity at the V1 branch of the trigeminal nerve.

In one embodiment the pressure adjustment is performed during an event in which there is a parasympathaetic influence on the autonomic nervous system, by which the trigeminal nerve is predisposed to the pressure adjustment and uptake of substance is increased.

In one embodiment the pressure is increased in the pressure adjustment step.

In one embodiment the method further comprises the step of: adjusting a concentration of NO in the nasal cavity before, during or after the delivery of the substance, whereby a rate of uptake of the substance is increased.

In one embodiment the NO concentration is adjusted by delivery of at least one gas.

In one embodiment the at least one gas is delivered in a flow, optionally having a concentration of at least 5 vol% of the at least one gas.

In one embodiment, the at least one gas comprises carbon dioxide.

In one embodiment adjustment of the NO concentration mediates activity at the V1 branch of the trigeminal nerve.

In one embodiment the NO concentration is decreased in the NO concentration adjustment step,

In one embodiment the substance is a substance which does not pass the blood-to-brain barrier.

In one embodiment the substance is a triptan. In one embodiment, the substance is sumatriptan.

In one embodiment the method is for the treatment of a neurological or CNS disorder.

In one embodiment, the method is for the treatment of headache, including cluster headache and migraine.

In one embodiment the method further comprises the step of: closing the oropharyngeal velum of the subject during delivery of the substance and/or the at least one gas.

In one embodiment the method further comprises the step of: the subject exhaling through a mouthpiece to cause closure of the oropharyngeal velum of the subject.

In one embodiment the mouthpiece is fluidly connected to a nosepiece, whereby exhaled air from an exhalation breath is delivered through the nosepiece.

In another aspect the present disclosure provides a method of administering a substance to a subject, comprising the steps of: delivering a substance to a posterior region of the nasal cavity of the subject, the posterior region comprising mucosa innervated by a trigeminal nerve; adjusting the pressure in the nasal cavity before, during or after the delivery of the substance, whereby a rate of uptake of the substance is increased.

In one embodiment the mucosa is further innervated by the sphenopalatine ganglion.

In one embodiment the substance is delivered through a nosepiece fitted to a nostril, optionally being a fluid-tight seal with a nare of the nostril.

In one embodiment, the substance is delivered through a single nostril to the mucosa one trigeminal nerve.

In one embodiment the substance is delivered successively through each of the nostrils to the mucosa at each of the trigeminal nerves.

In one embodiment the pressure is adjusted by delivery of at least one gas.

In one embodiment the at least one gas is delivered in a flow, optionally having a concentration of at least 5 vol% of the at least one gas.

In one embodiment, the at least one gas comprises carbon dioxide.

In one embodiment, adjustment of the pressure mediates activity at the V1 branch of the trigeminal nerve.

In one embodiment the pressure adjustment is performed during an event in which there is a parasympathaetic influence on the autonomic nervous system, by which the trigeminal nerve is predisposed to the pressure adjustment and uptake of substance is increased.

In one embodiment the pressure Is at least about 3 kPa, optionally from about 3 to about 7 kPa.

In one embodiment the pressure is increased in the pressure adjustment step.

In one embodiment the method further comprises the step of: adjusting a concentration of NO in the nasal cavity before, during or after the delivery of the substance, whereby a rate of uptake of the substance is increased.

In one embodiment the NO concentration is adjusted by delivery of at least one gas.

In one embodiment the at least one gas is delivered in a flow, optionally having a concentration of at least 5 vol% of the at least one gas.

In one embodiment the at least one gas comprises carbon dioxide.

In one embodiment adjustment of the NO concentration mediates activity at the V1 branch of the trigeminal nerve.

In one embodiment the NO concentration is decreased in the NO concentration adjustment step.

In one embodiment the substance is a substance which does not pass the blood-to-brain barrier.

In one embodiment the substance is a triptan. In one embodiment, the substance is sumatriptan.

In one embodiment the method is used in the treatment of a neurological or CNS disorder. In one embodiment in the treatment of headache, including cluster headache and migraine.

In one embodiment the method further comprises the step of: closing the oropharyngeal velum of the subject during delivery of the substance and/or the at least one gas.

In one embodiment the method further comprises the step of: the subject exhaling through a mouthpiece to cause closure of the oropharyngeal velum of the subject.

In one embodiment the mouthpiece is fluidly connected to a nosepiece, whereby exhaled air from an exhalation breath is delivered through the nosepiece.

In a further aspect the present disclosure provides a method of administering a substance to a subject, comprising the steps of: delivering a substance to a posterior region of the nasal cavity of the subject, the posterior region comprising mucosa innervated by a trigeminal nerve; adjusting a concentration of NO in the nasal cavity before, during or after the delivery of the substance, whereby a rate of uptake of the substance in increased.

In one embodiment the mucosa is further innervated by the sphenopalatine ganglion.

In one embodiment the substance is delivered through a nosepiece fitted to a nostril, optionally being a fluid-tight seal with a nare of the nostril.

In one embodiment the substance is delivered through a single nostril to the mucosa one trigeminal nerve.

In one embodiment the substance is delivered successively through each of the nostrils to the mucosa at each of the trigeminal nerves.

In one embodiment the NO concentration is adjusted by delivery of at least one gas.

In one embodiment the at least one gas is delivered in a flow, optionally having a concentration of at least 5 vol% of the at least one gas.

In one embodiment the at least one gas comprises carbon dioxide.

In one embodiment adjustment of the NO concentration mediates activity at the V1 branch of the trigeminal nerve.

In one embodiment the NO concentration is decreased in the NO concentration adjustment step.

In one embodiment the method further comprises the step of: adjusting a pH of the mucosa, before, during or after the delivery of the substance, whereby a rate of uptake of the substance is increased.

in one embodiment the pH is adjusted by delivery of at least one gas.

In one embodiment the at least one gas is delivered in a flow, optionally having a concentration of at least 5 vol% of the at least one gas.

In one embodiment the at least one gas comprises carbon dioxide.

In one embodiment adjustment of the pH mediates activity at the V1 branch of the trigeminal nerve.

In one embodiment the pH adjustment is performed during an event in which there is a parasympathaetic influence on the autonomic nervous system, by which the trigeminal nerve is predisposed to the pH adjustment and uptake of substance is increased.

In one embodiment the pH is reduced in the pH adjustment step.

In one embodiment the method further comprises the step of: adjusting a pressure in the nasal cavity before, during or after the delivery of the substance, whereby a rate of uptake of the substance is increased.

In one embodiment the pressure is at least about 3 kPa. optionally from about 3 to about 7 kPa.

In one embodiment the pressure is adjusted by delivery of at least one gas.

In one embodiment the at least one gas is delivered in a flow, optionally having a concentration of at least 5 vol% of the at least one gas.

In one embodiment the at least one gas comprises carbon dioxide.

In one embodiment the pressure adjustment mediates activity at the V1 branch of the trigeminal nerve.

In one embodiment the pressure adjustment is performed during an event in which there is a parasympathaetic influence on the autonomic nervous system, by which the trigeminal nerve is predisposed to the pressure adjustment and uptake of substance is increased.

In one embodiment the pressure is increased in the pressure adjustment step.

In one embodiment the substance is a substance which does not pass the blood-to-brain barrier.

In one embodiment the substance is a triptan, In one embodiment the substance is sumatriptan.

In one embodiment the method is used in the treatment of a neurological or CNS disorder, in one embodiment in the treatment of headache, including cluster headache and migraine.

In one embodiment the method further comprises the step of: closing the oropharyngeal velum of the subject during delivery of the substance and/or the at least one gas.

In one embodiment the method further comprises the step of: the subject exhaling through a mouthpiece to cause closure of the oropharyngeal velum of the subject.

In one embodiment the mouthpiece is fluidly connected to a nosepiece, whereby exhaled air from an exhalation breath is delivered through the nosepiece.

In a still further aspect the present disclosure provides a method of administering a substance to a subject, comprising the steps of: delivering a substance to a posterior region of the nasal cavity of the subject, the posterior region comprising mucosa innervated by a trigeminal nerve; adjusting a pH of the mucosa before, during or after the delivery of a substance; and adjusting a pressure In the nasal cavity before, during or after the delivery of the substance, whereby a rate of uptake of the substance is increased.

In yet another aspect the present disclosure provides a method of administering a substance to a subject, comprising the steps of: delivering a substance to a posterior region of the nasal cavity of the subject, the posterior region comprising mucosa innervated by a trigeminal nerve; adjusting a pH of the mucosa before, during or after the delivery of a substance; and adjusting a concentration of NO in the nasal cavity before, during or after the delivery of the substance, whereby a rate of uptake of the substance is increased.

In still another aspect the present disclosure provides a method of administering a substance to a subject, comprising the steps of: delivering a substance to a posterior region of the nasal cavity of the subject, the posterior region comprising mucosa innervated by a trigeminal nerve; adjusting a pressure in the nasal cavity before, during or after the delivery of the substance; and adjusting a concentration of NO in the nasal cavity before, during or after the delivery of the substance, whereby a rate of uptake of the substance in increased.

In still another aspect the present disclosure provides a method of administering a substance to a subject, comprising the steps of: delivering a substance to a posterior region of the nasal cavity of the subject, the posterior region comprising mucosa innervated by a trigeminal nerve; adjusting a pH of the mucosa before, during or after the delivery of the substance; and adjusting a pressure in the nasal cavity before, during or after the delivery of the substance; and adjusting a concentration of NO in the nasal cavity before, during or after the delivery of the substance, whereby a rate of uptake of the substance is increased.

In a yet further aspect the present disclosure provides a substance for treating a neurological or CNS disorder, wherein the substance is delivered to a posterior region of the nasal cavity of a subject, the posterior region comprising mucosa innervated by a trigeminal nerve; and wherein a pH of the mucosa is adjusted before, during or after the delivery of the substance, whereby a rate of uptake of the substance is increased.

In a still yet further aspect the present disclosure provides substance for treating a neurological or CNS disorder, wherein the substance is delivered to a posterior region of the nasal cavity of a subject, the posterior region comprising mucosa innervated by a trigeminal nerve; and wherein a pressure in the nasal cavity is adjusted before, during or after the delivery of the substance, whereby a rate of uptake of the substance is increased.

In yet still another aspect the present disclosure provides a substance for treating a neurological or CNS disorder, wherein the substance is delivered to a posterior region of the nasal cavity of a subject, the posterior region comprising mucosa Innervated by a trigeminal nerve; and wherein a concentration of NO in the nasal cavity is adjusted before, during or after the delivery of the substance, whereby a rate of uptake of the substance is increased.

In one embodiment the substance is a triptan. In one embodiment, the substance is sumatriptan.

In one aspect the substance is for the treatment of headache, including cluster headache and migraine.

In a further aspect the present disclosure provides a method of administering a substance to a subject, comprising the steps of delivering a substance to a subject; adjusting a pressure in the nasal cavity before, during or after the delivery of the substance, whereby a rate of uptake of the substance is increased.

In yet another aspect the present disclosure provides a method of administering a substance to a subject, comprising the steps of delivering a substance to a subject; adjusting a concentration of NO in the nasal cavity before, during or after the delivery of the substance, whereby a rate of uptake of the substance is increased.

In a yet further aspect the present disclosure provides a method of administering a substance to a subject, comprising the steps of delivering a substance to a subject; adjusting a pH of the mucosa innervated by a trigeminal nerve before, during or after the delivery of the substance, whereby a rate of uptake of the substance is increased.

In one embodiment the delivery is peroral, topical, transmucosal, inhalation and/or injection, subcutaneous, nasal, and/or oral.

In a further aspect the present disclosure provides a method of administering a substance to a subject, comprising the steps of delivering a first substance that induces a migraine; and delivering a second substance according to any of the methods disclose above.

In accordance with the disclosure, an embodiment is directed to a method of therapeutically treating a patient. The method can include administering, in a first step, a therapeutic agent. The method can also include delivering, in a second step, to a location at an interior of a nasal passage of the patient a therapeutic amount of at least one of carbon dioxide or a pH adjusting material.

Another embodiment is directed to a method for Increasing a therapeutic effect of a pharmaceutical agent delivered to a patient. The method can include delivering a fluid flow to a nasal passage of the patient to deliver about 5% to about 6% vol/vol carbon dioxide to an upper posterior region of the nasal passage. The method can also include administering a dose of the pharmaceutical agent to the patient.

Yet another embodiment is directed to a method of treating a patient that includes delivering about 5% to about 6% vol/vol carbon dioxide to a nostril of the patient to lower a pH of an upper posterior region of the nasal passage by at least about 0.1 pH units to provide a therapeutic effect.

Additional objects and advantages of the disclosure will be set forth in part in the description which follows, and in part will be obvious from the description, or may be learned by practice of the disclosure. The objects and advantages of the disclosure will be realized and attained by means of the elements and combinations particularly pointed out In the appended claims.

It is to be understood that both the foregoing general description and the following detailed description are exemplary and explanatory only and are not restrictive of the disclosure, as claimed.

The accompanying drawings, which are incorporated in and constitute a part of this specification, together with the description disclose embodiments of the invention, which are by way of example only and serve to explain the principles of the disclosure.

### BRIEF DESCRIPTION OF THE DRAWINGS

Figure 1(a) schematically illustrates the anatomy of the upper respiratory tract of a human subject;
Figure 1(b) illustrates the segmentation of a nasal cavity in accordance with an embodiment of the present disclosure;
Figures 2(a) and (b) illustrate a nasal delivery device in accordance with one embodiment of the present disclosure;
Figures 3(a) and (b) illustrate a nasal delivery device in accordance with another embodiment of the present disclosure;
Figure 4 illustrates the response rates for Example #1;
Figure 5 illustrate the pharmacokinetic parameters calculated in Example #2;
Figure 6 illustrates sumatriptan plasma concentration-time profiles over a 14 hour sampling period for intranasal sumatriptan powder, 20 mg nasal spray, 100 mg tablet and 6 mg subcutaneous injection and inset for intranasal sumatriptan powder, 20 mg nasal spray and 100 mg tablet over the first 30 minutes post-dose, for Example #2;

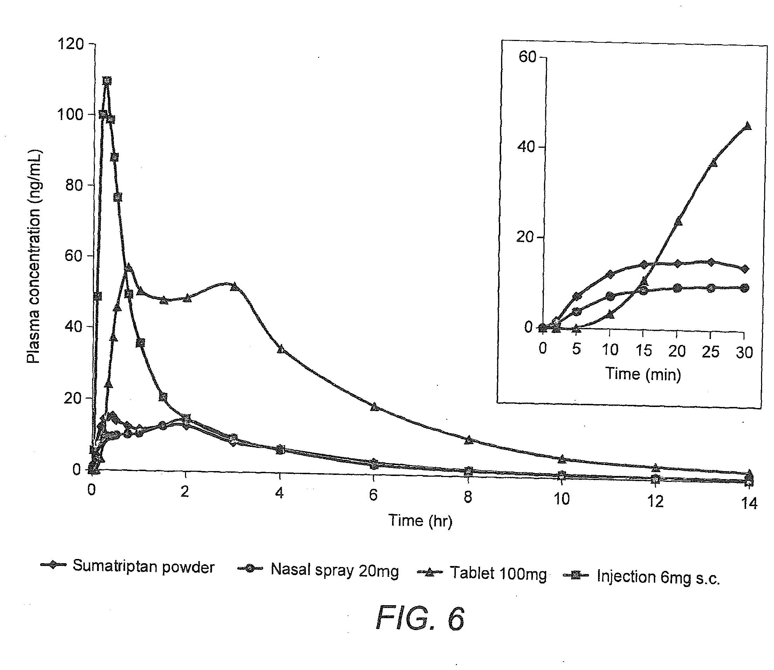

The main figure in Figure 6 shows that both methods of intranasal delivery resulted in much lower mean plasma sumatriptan concentration-time profiles than observed for the tablet and the injection. The inset in Figure 6 illustrates in the first 30 minutes post-dose, the rate of rise of plasma sumatriptan concentration was faster for sumatriptan powder than either the 20 mg nasal spray or the 100 mg tablet.

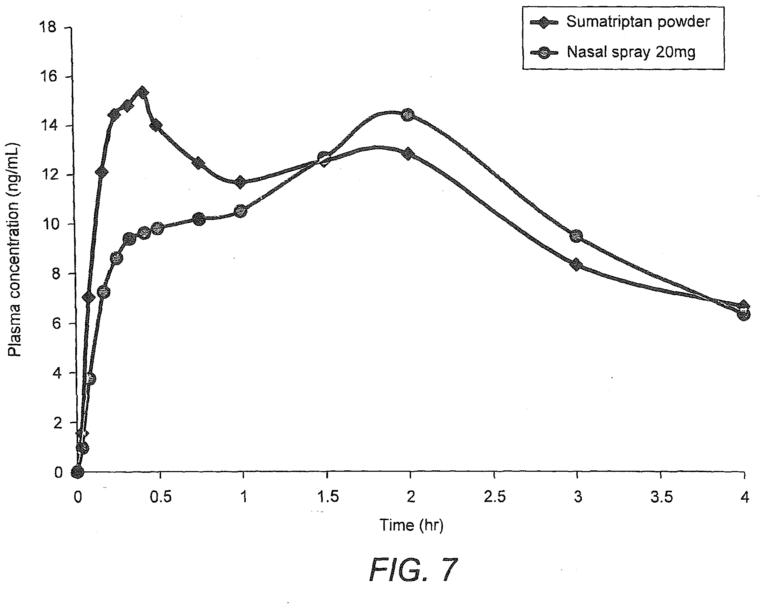

Figure 7 illustrates sumatriptan plasma concentration-time profiles over the first 4 hours after administration of sumatriptan powder by the device of the present disclosure as compared with the 20 mg nasal spray for Example #2;

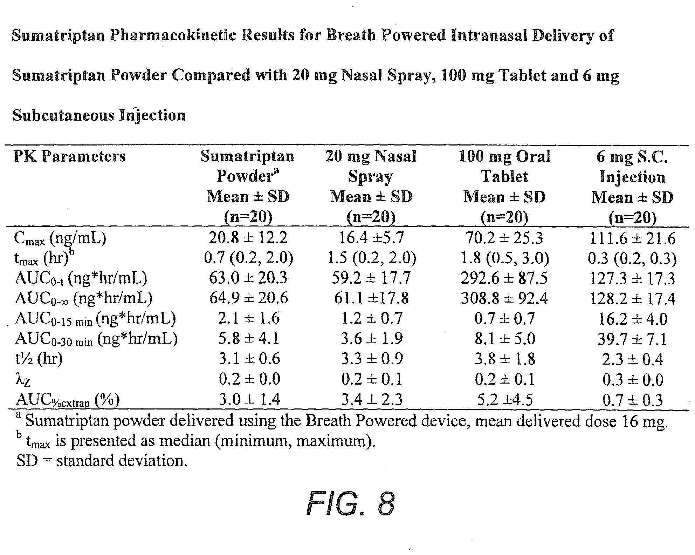

Figure 8 illustrates sumatriptan pharmacokinetic results for breath powered intranasal delivery of sumatriptan powder compared with 20 mg nasal spray, 100 mg tablet and 6 mg subcutaneous injection for Example #2;

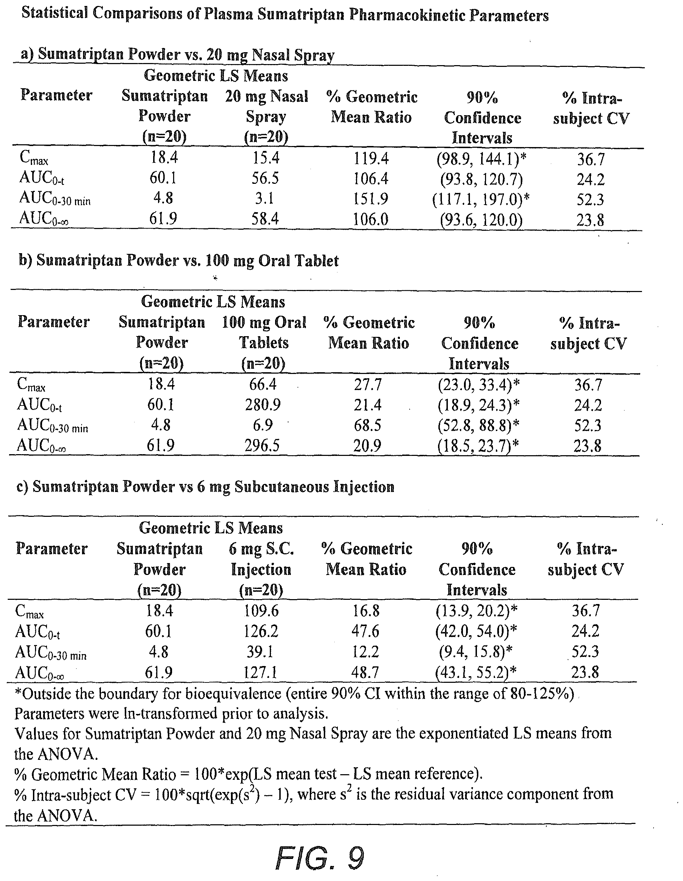

Figure 9 illustrates statistical comparisons of plasma sumatriptan pharmacokinetic parameters for Example #2;

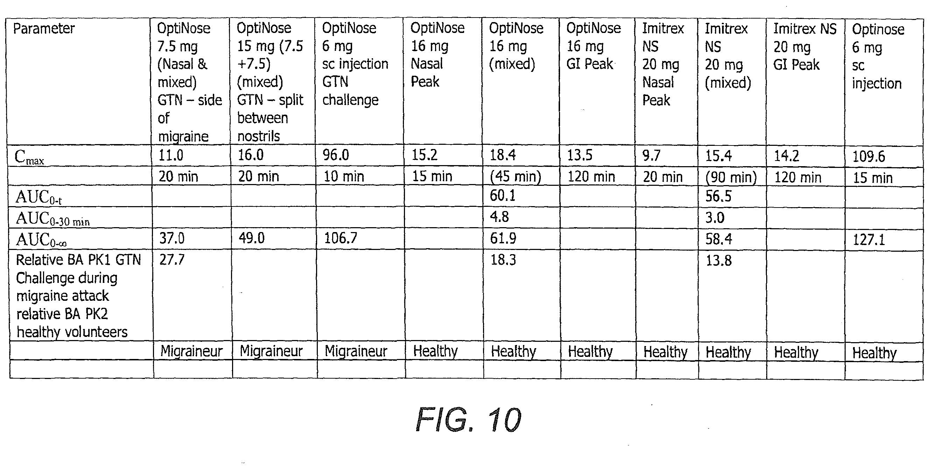

Figure 10 illustrates statistical comparisons of sumatriptan plasma pharmacokinetic parameters, including for nitroglycerin (GTN)-induced migraines and on healthy subjects for Example #3.

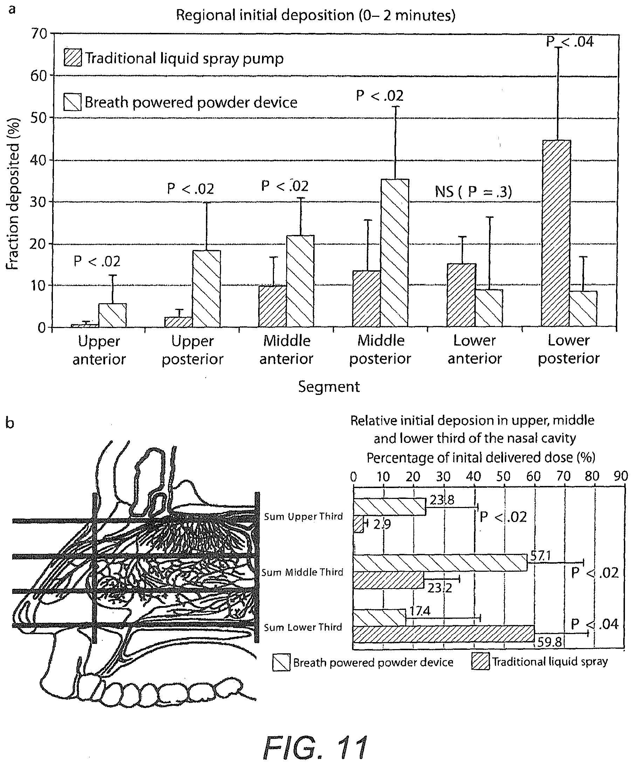

Figure 11(a) shows initial regional nasal deposition (0-2 mins) for breath powered powder delivery device and delivery with a traditional nasal spray pump.

Figure 11(b) shows initial horizontal nasal distribution (0-2 mins) for breath powered powder delivery device and delivery with a conventional nasal spray pump.

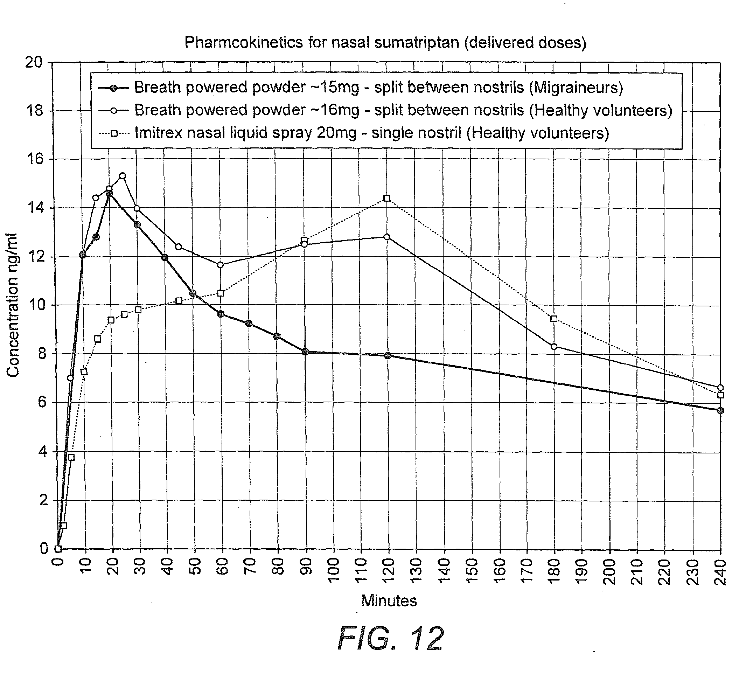

Figure 12 shows pharmacokinetics (PK) profiles for nasal sumatriptan from two crossover studies performed with the Breath Powered powder device and the marketed Imitrex sumatriptan nasal spray. The one study was done in migraine patients during GTN challenge, whereas the other study was performed in healthy volunteers.

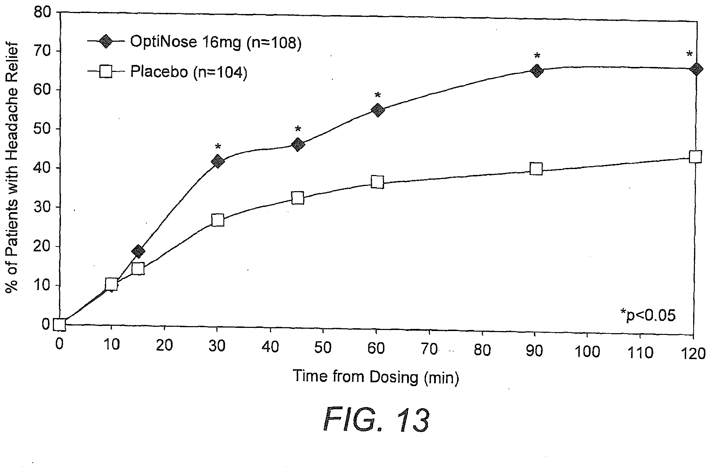

Figure 13 shows percent of patients with headache relief.

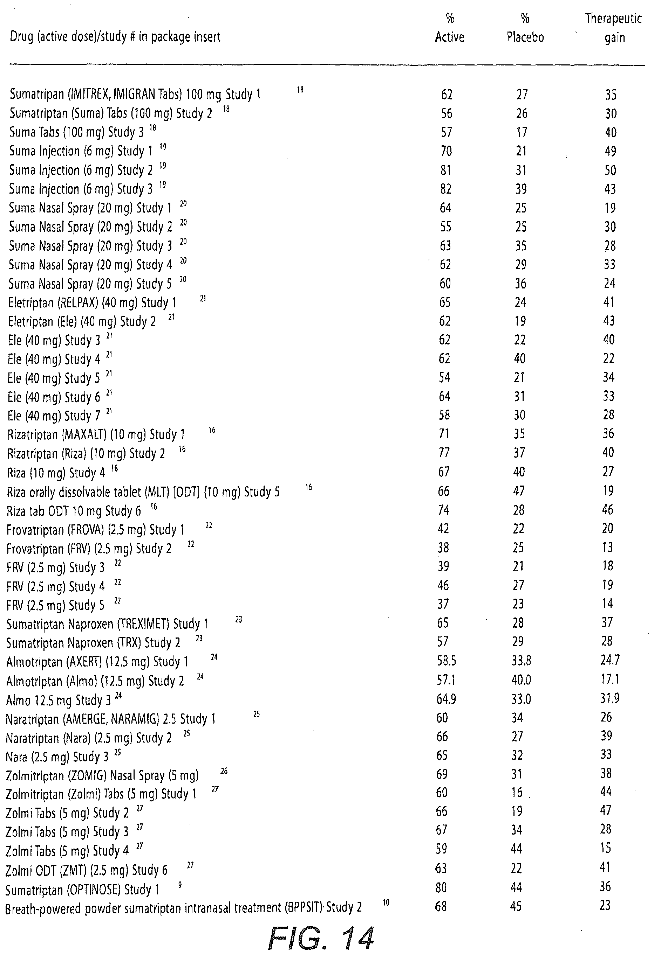

Figure 14 shows two-hour pain relief as reported in package inserts.

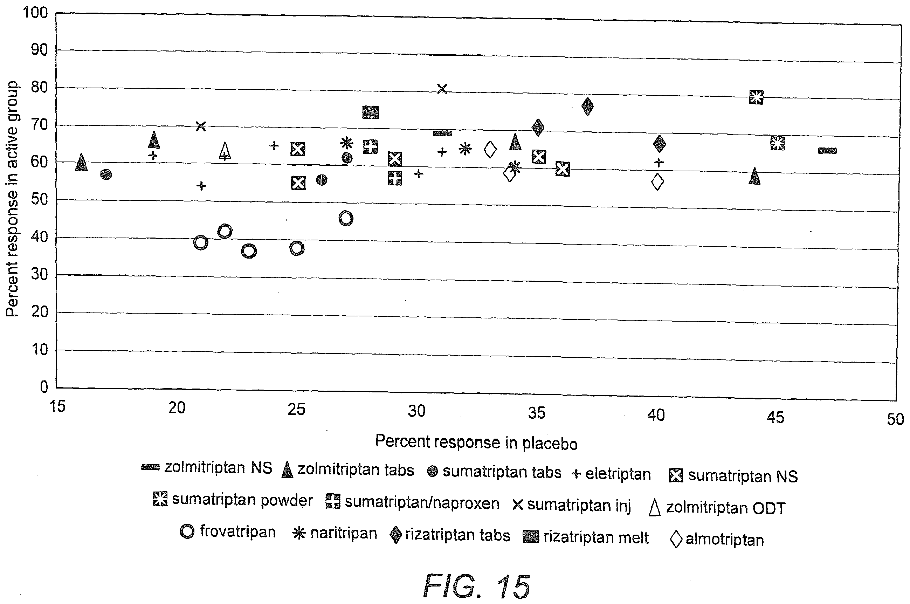

Figure 15 shows two-hour pain response rates reported in package inserts by study for active and placebo.

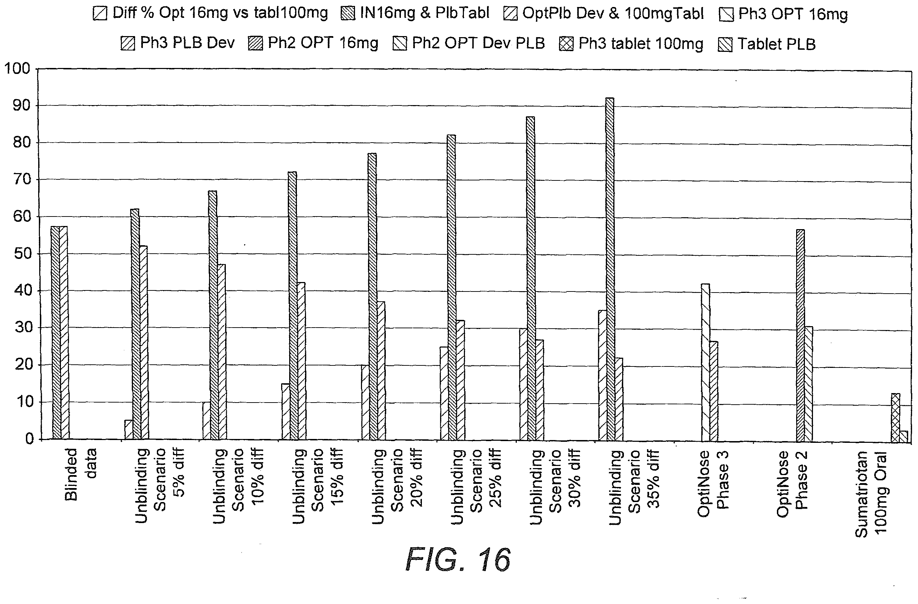

Figure 16 shows "blinded" data from March 2014.

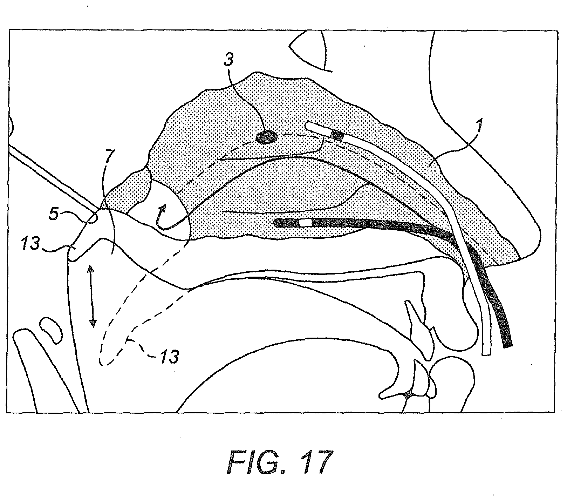

Figure 17 shows a pH probe located generally at upper and lower regions of a nasal passage.

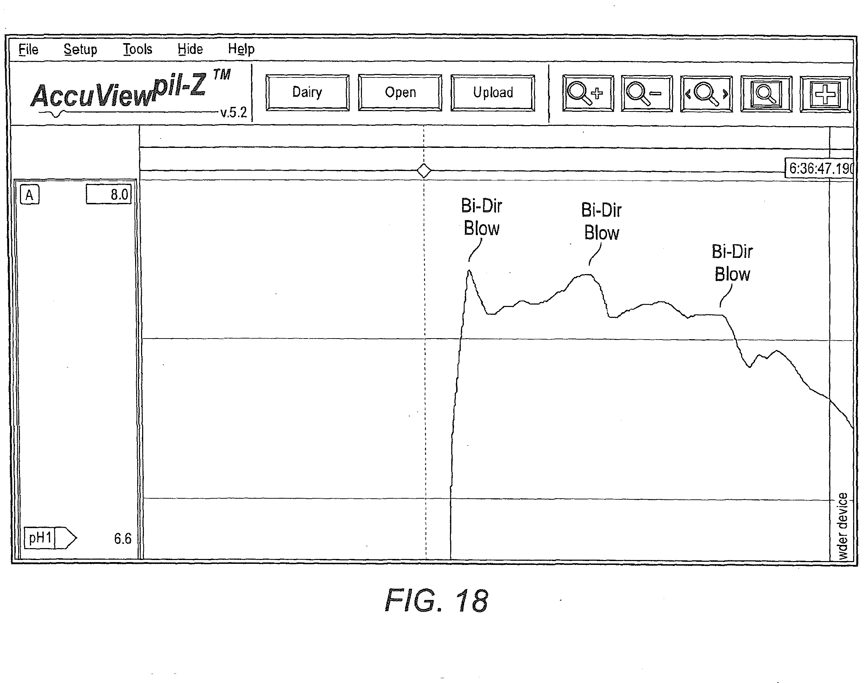

Figure 18 shows data gathered from a pH probe located generally at the nasal roof and on the same side as an inhalation device.

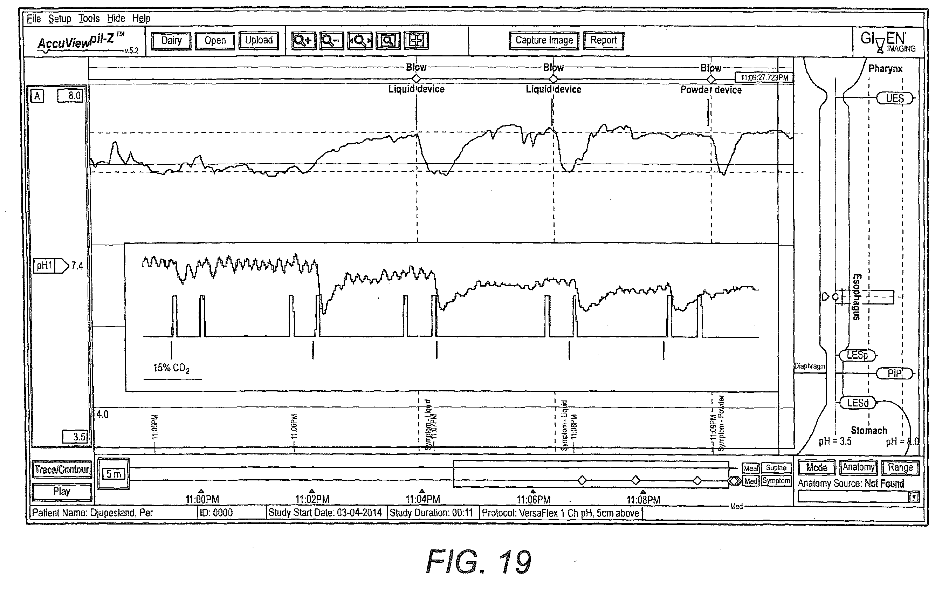

Figure 19 shows data gathered from a pH probe located generally at the nasal roof and about 4 - 5 cm from a nostril opening. Data for liquid and powder delivery devices are shown.

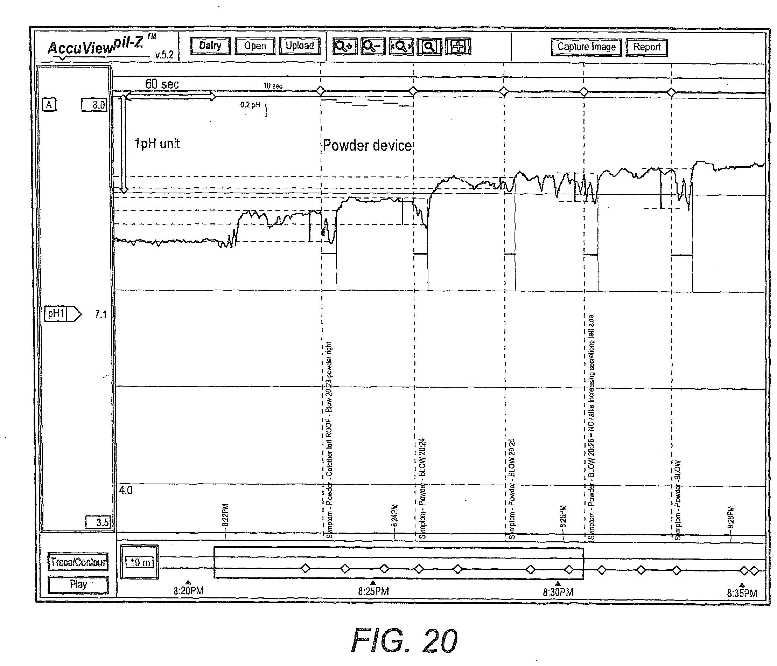

Figure 20 show data associated with powder delivery, with a sensor located about 4 - 5 cm into a nasal passage at the floor/middle part of the passage.

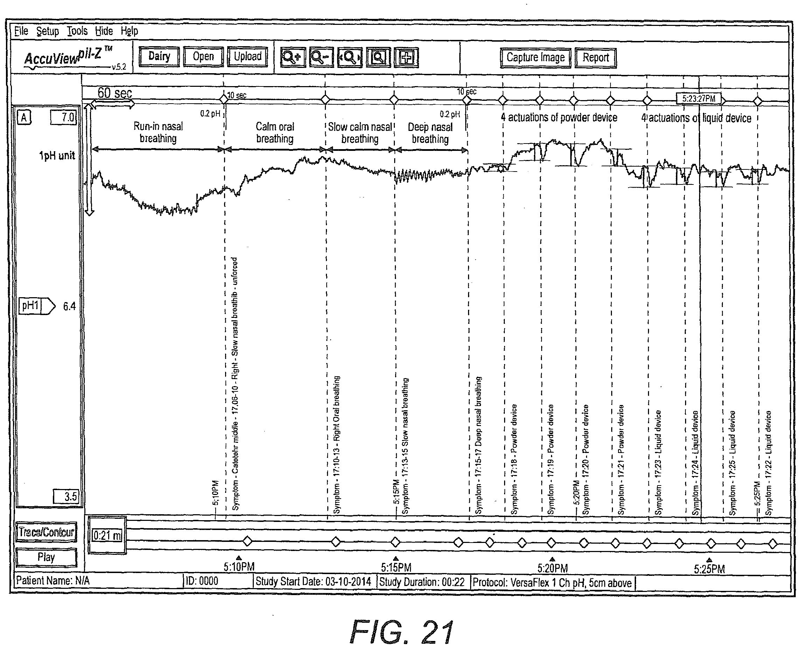

Figure 21 show data associated with powder delivery, with a sensor located about 4 - 5 cm into a nasal passage at the floor/middle part of the passage.

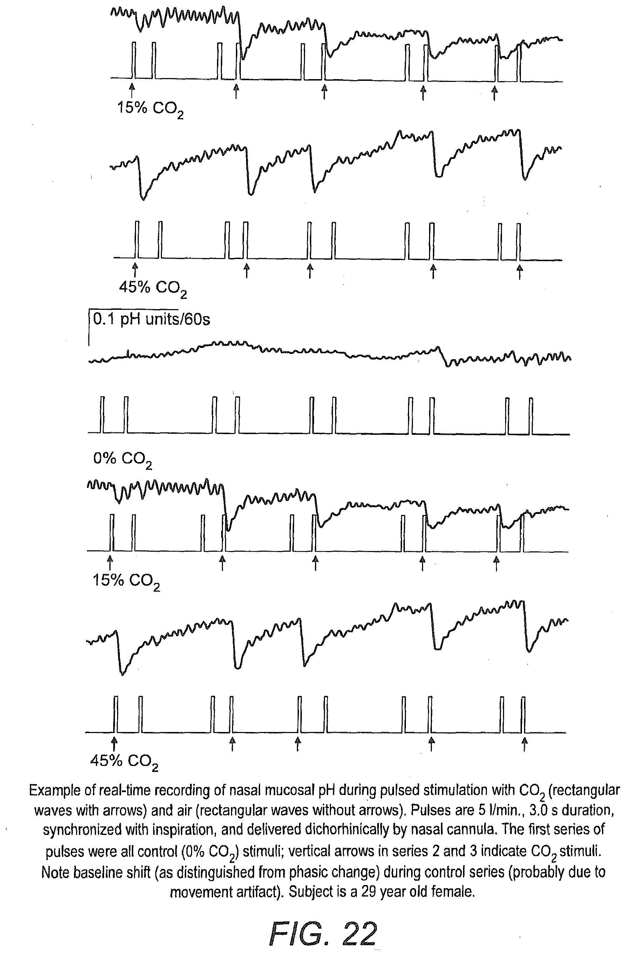

Figure 22 shows data from a prior art reference.

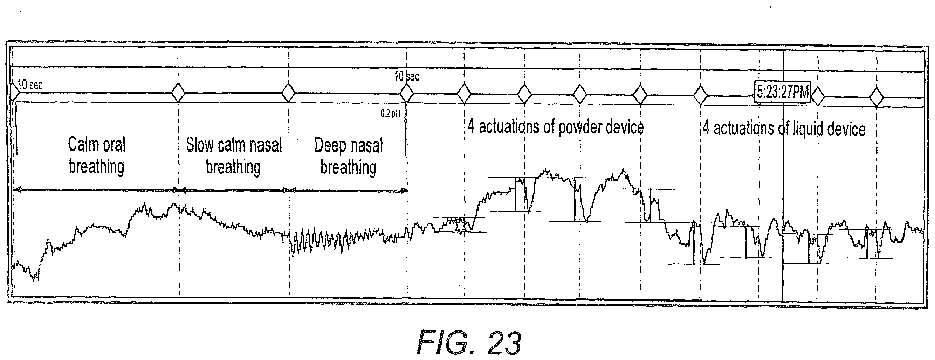

Figure 23 shows data associated with the inhalation device described herein.

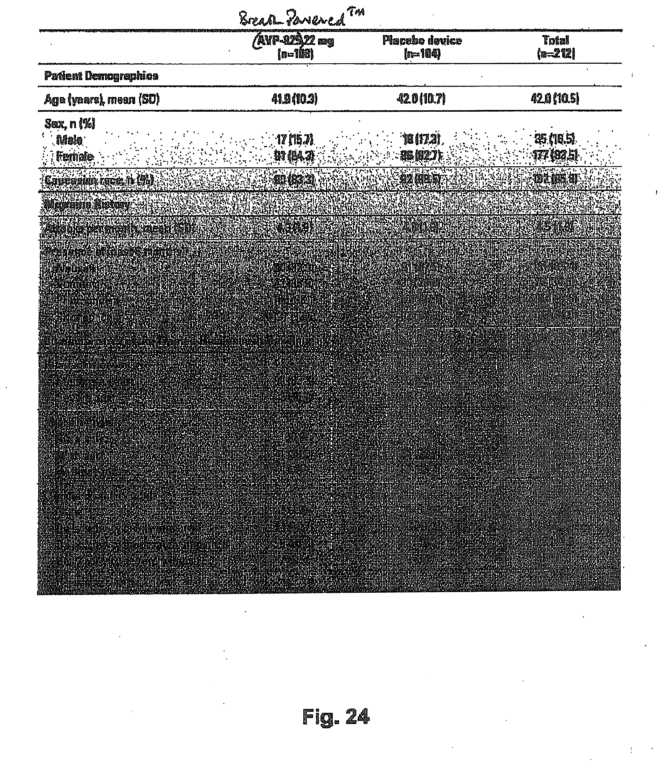

Figure 24 shows patient demographics and baseline characteristics (FAS).

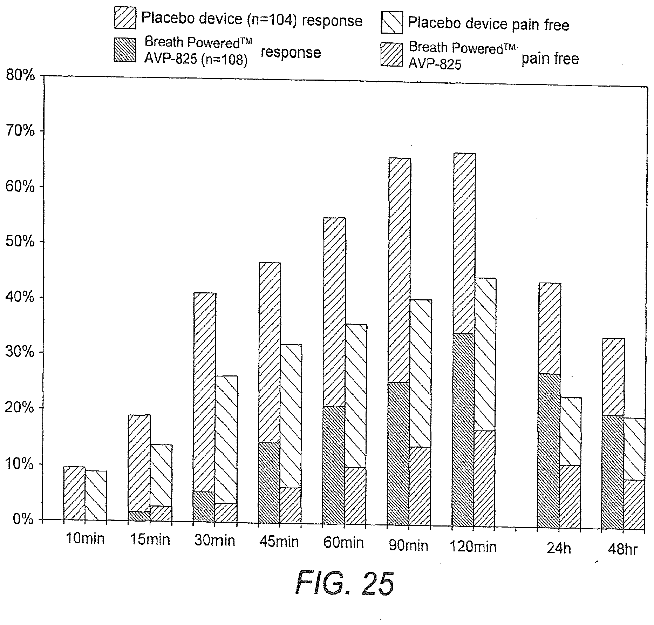

Figure 25 shows a distribution of data associated with the breach powered inhalation device and placebo data.

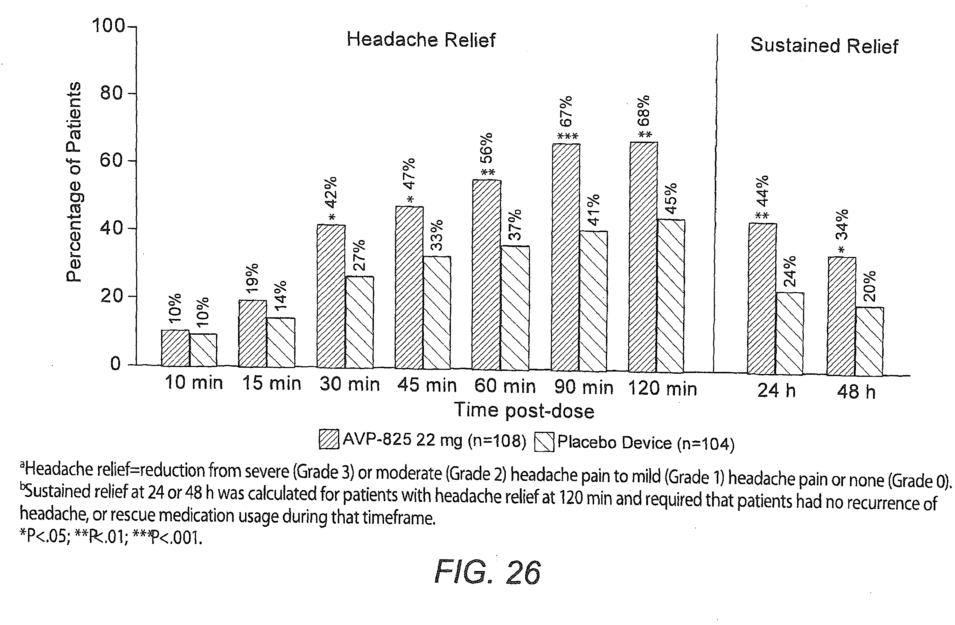

Figure 26 shows the proportion of patients with headache relief ^{a} at protocol specified time points up to 120 min post-dose and who sustained relief ^{b} at 24 and 48 h (FAS).

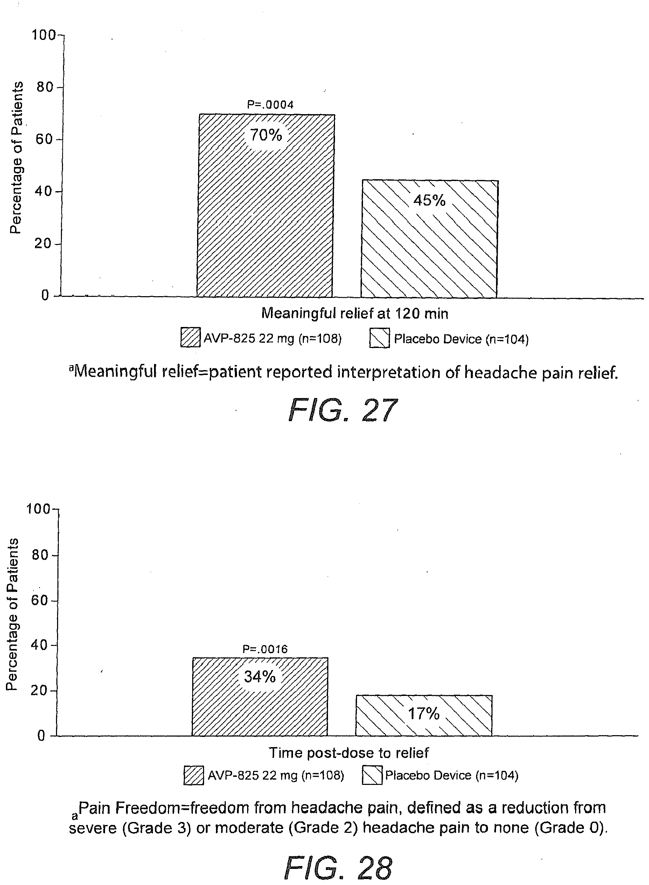

Figure 27 shows a proportion of patients with meaningful relief ^{a} following treatment with AVP-825 or placebo device at 120 min post-dose (FAS).

Figure 28 shows proportion of patients who achieved pain freedom at 120 min endpoint (FAS).

### DESCRIPTION OF EMBODIMENTS

Reference will now be made in detail to the exemplary embodiments of the disclosure, examples of which are illustrated in the accompanying drawings. Wherever possible, the same reference numbers will be used throughout the drawings to refer to the same or like parts.

### Exemplarary Delivery Devices

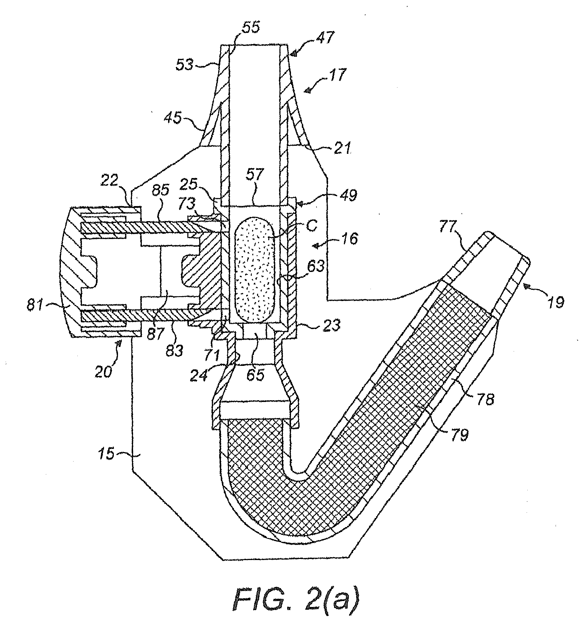

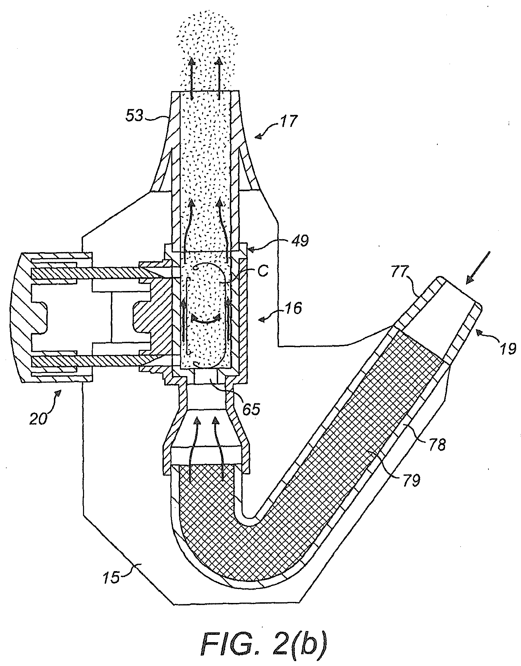

Figures 2(a) and (b) illustrate a Breath Powered^{™} powder delivery device which is operative to deliver a powder aerosol.

The Breath Powered^{™} delivery device comprises a housing 15, a capsule-receiving unit 16 for receiving a capsule C, a nosepiece unit 17 for fitting to a nasal cavity of a subject, a mouthpiece unit 19 through which the subject exhales, and a capsule-piercing mechanism 20, which is operable to pierce a capsule C as contained by the capsule-receiving unit 16 and thereby prime the delivery device for operation.

The housing 15 includes a first, nosepiece aperture 21, in this embodiment at the upper end of the housing 15, which receives the nosepiece unit 17, and a second, lateral aperture 22, in this embodiment in an end wall of the housing 15, through which extends an actuator button 81 of the capsule-piercing mechanism 20, as will be described in more detail herein.

The capsule-receiving unit 16 comprises a capsule-receiving member 23, in this embodiment an elongate, upstanding chamber which is disposed opposite the nosepiece aperture 21 in the housing 15, for receiving a capsule C, in this embodiment as contained within a capsule-containing member 49 of the nosepiece unit 17, as will be described in more detail herein.

In this embodiment the capsule-receiving member 23 includes an inlet 24 and an outlet 25 for providing for an air flow therethrough, with the outlet 25, as defined by an upper, downstream end of the capsule-receiving member 23, being adapted to receive the capsule-containing member 49 of the nosepiece unit 17, such that the capsule-containing member 49 is a sealing fit within the capsule-receiving member 23.

The nosepiece unit 17 comprises a main body member 45 which is configured to fit in the nosepiece aperture 21 of the housing 15, a nosepiece 47 which extends outwardly of the main body member 45 for fitting to the nostril of the subject, and a capsule-containing member 49 which extends inwardly of the main body member 45 and contains a capsule C, the contents of which are to be delivered to the nasal cavity of the subject. In this embodiment the capsule C is a hydroxypropyl methylcellulose (HPMC) capsule which contains a particulate substance, such as a powdered substance, and typically a pharmaceutical substance. In other embodiments the capsule C could be formed substantially of another cellulose derivative, such as hydroxypropylcellulose, methylcellulose, ethylcellulose and carboxymethylcellulose. In an alternative embodiment the capsule C can be formed from a gelatine derivative. In one embodiment the capsule C can be coated with a hydrophobic material, such as parylene.

In this embodiment the nosepiece 47 has a substantially frusto-conical outer section 53 for guiding the nosepiece unit 17 into a nasal passage of the subject and providing a fluid-tight seal with the nares of the nostril, and includes an inner channel 55, here of substantially cylindrical section, through which substance is delivered to a posterior region of the nasal passage of the subject, in this embodiment an upper posterior region as bounded by a vertical plane which is located posterior of the anterior nasal spine AnS at a position corresponding to one-quarter of the distance between the anterior and posterior nasal spines AnS, PnS and a horizontal plane which is located above the nasal floor at a height one-third of the distance between the nasal floor and the cribiform plate. As discussed hereinabove, the present inventors have recognized that an increased delivery of powdered substance to the upper posterior region of the nasal passage surprisingly provides for a very rapid onset of action as compared to the conventional nasal administration of a liquid substance.

In this embodiment the nosepiece 47 is configured to deliver a significant fraction of substance to the upper posterior region of the nasal passage, here an initial deposition of greater than 30% of the delivered dose.

In this embodiment the nosepiece 47, in providing a fluid-tight seal with the nostril of the subject, provides for bi-directional delivery through the nasal airway of the subject, as disclosed in the applicant's earlier WO-A-2000/051672, which is incorporated by reference in its entirety. In another embodiment, however, the nosepiece 47 need not provide a sealing fit, thus encompassing delivery to the nasal cavity, but not necessarily bi-directional delivery.

In this embodiment the nosepiece 47 includes a trap element 57, typically a perforated or mesh element, for preventing any foreign matter, such as a part of the capsule C, which is above a predetermined size from passing through the nosepiece 47 and into the nasal cavity of the subject.

The capsule-containing member 49 includes an elongate flow passage 63, in this embodiment cylindrical in shape, in which the capsule C is oriented axially therealong such as to be rotatable therewithin when an air flow is delivered therethrough, and an inlet aperture 65 in fluid communication with one, the downstream, end of the flow passage 63, which inlet aperture 65 provides a flow restriction to an air flow as delivered therethrough and acts as a seat for one, the lower, end of the capsule C prior to the delivery of an air flow through the flow passage 63.

The capsule-containing member 49 further includes a plurality of, in this embodiment first and second piercing apertures 71, 73 in a lateral wall thereof for enabling the capsule C to be pierced at locations spaced along the axial length thereof. In this embodiment the first, lower aperture 71 is located such that the capsule C is pierced at a location above the height of the dose of substance as contained thereby when the lower end of the capsule C is seated in the inlet aperture 65 of the flow passage 63. In this way, the dose of substance as contained by the capsule C is not released into the flow passage 63 until an air flow is delivered through the flow passage 63.

In this embodiment the nosepiece unit 17 is provided as a replaceable unit which is replaced following each operation of the delivery device. In this embodiment the nosepiece unit 17 can be packaged in air-tight packaging, for example, an aluminum foil package.

The mouthpiece unit 19 comprises a mouthpiece 77, in this embodiment as gripped in the lips of the subject, through which the subject exhales to deliver an entraining air flow through the capsule-receiving unit 16, and an air chamber 78, in this embodiment an elongate tubular section, which fluidly connects the mouthpiece 77 and the capsule-receiving unit 16.

In this embodiment the air chamber 78 has a greater volume than the capsule-receiving member 23 of the capsule-receiving unit 16, and in one embodiment has a volume at least twice that of the capsule-receiving member 23.

In this embodiment the air chamber 78 incorporates a temperature regulator 79, here formed as a condenser for cooling the exhaled air flow, at least at the upstream end thereof. With this configuration, the exhaled air flow is cooled during exhalation.

In this embodiment the temperature regulator 79 comprises a labyrinthine structure. In another embodiment the temperature regulator 79 could be provided by a filter element, which could also act as a microbiological filter.

In one embodiment the temperature regulator 79 could include means for drying the condensate as collected therein when the delivery device is not in use.

In one embodiment the air chamber 78 is removable, such as to allow for cleaning or replacement.

This arrangement has been found to provide for reliable operation of the delivery device, in delivering substance from the capsule C. The present inventors have established that the provision of moist exhaled air directly to the capsule C can sometimes prevent the required rotation of the capsule C, and thereby prevent proper release of the substance as contained thereby. By providing a volume of cooler air, and arranging for that volume of cooler air to be delivered initially in a burst, the required rotation of the capsule C is seen repeatedly.

The capsule-piercing mechanism 20 comprises an actuator button 81 which extends through the lateral aperture 22 in the housing 15 such as to allow for operation by the subject, a plurality of, in this embodiment first and second piercing elements 83, 85 which are supported by the actuator button 81 and extend forwardly thereof, such that, on depression of the actuator button 81 from a retracted position to an extended position, the piercing elements 83, 85 are driven through respective ones of the piercing apertures 71, 73 in the lateral wall of the capsule-containing member 49 to pierce the capsule C.

In this embodiment the capsule-piercing mechanism 20 includes a resilient element 87 which acts to bias the actuator button 81 outwardly towards the retracted position, such that, following depression of the actuator button 81 to pierce the capsule C, the actuator button 81 is returned to the retracted position. In this embodiment the resilient element 87 is formed as an integral part of the actuator button 81, but in other embodiments could be provided by a separate element, such as a compression spring.

Operation of the delivery device is as follows.

Firstly, taking the delivery device in hand, and with a nosepiece unit 17 inserted in the housing 15, the subject depresses the actuator button 81 of the capsule-piercing mechanism 20 such as to pierce the capsule C as contained in the capsule-containing member 49.

By depressing the actuator button 81, the capsule C is pierced by the piercing elements 83, 85 at two locations spaced along the axial length thereof.

In this embodiment the first, lower piercing element 83 acts to pierce the capsule C at a location just above the height of the substance as contained by the capsule C, the capsule C only being part filled, and the second, upper piercing element 85 acts to pierce the upper, distal end of the capsule C.

The actuator button 81 is then released, which causes the actuator button 81 to be returned to the retracted position under the bias of the biasing element 87. In this way, the delivery device is primed and ready for use.

The subject then inserts the nosepiece 47 into one of his/her nasal passages until the nosepiece 47 abuts the nares of the nostril such as to establish a fluid-tight seal therewith, at which point the distal end of the nosepiece 47 extends about 2 cm into the nasal passage of the subject, and grips the mouthpiece 77 in his or her lips.

The subject then begins to exhale through the mouthpiece 47, which exhalation acts to close the oropharyngeal velum of the subject and drive an air flow through the nasal airway of the subject, with the air flow passing into the one nasal passage, around the posterior margin of the nasal septum and out of the other nasal passage, thereby achieving a bi-directional air flow through the nasal airway of the subject.

When the subject exhales with sufficient force, the capsule C is lifted from the seat as defined by the inlet aperture 65 of the capsule-containing member 49 and the capsule C is rotated, which rotation acts to release the substance from within the capsule C which is entrained by the exhaled air flow and delivered to the posterior region of the nasal cavity of the subject. With continued exhalation, the capsule C continues to rotate.

Further, in this device, the capsule C is configured to vibrate, and through the sound transmission path as provided by the nosepiece unit 17 being inserted into the nostril, this vibration acts to promote ventilation of the nasal airway, particularly In the posterior region of the nasal cavity. It Is postulated that this vibration contributes to efficacy, as outlined in the studies described below.

This operation of the delivery device can be repeated with a new capsule C. In this embodiment the entire nosepiece unit 17 is replaced, but in other embodiments either the capsule-containing member 49 or just the capsule C could be replaced.

The gas may be delivered at a pressure of 2, 3, 4, 5, 6, 7, 8, 9 or 10 kPa.

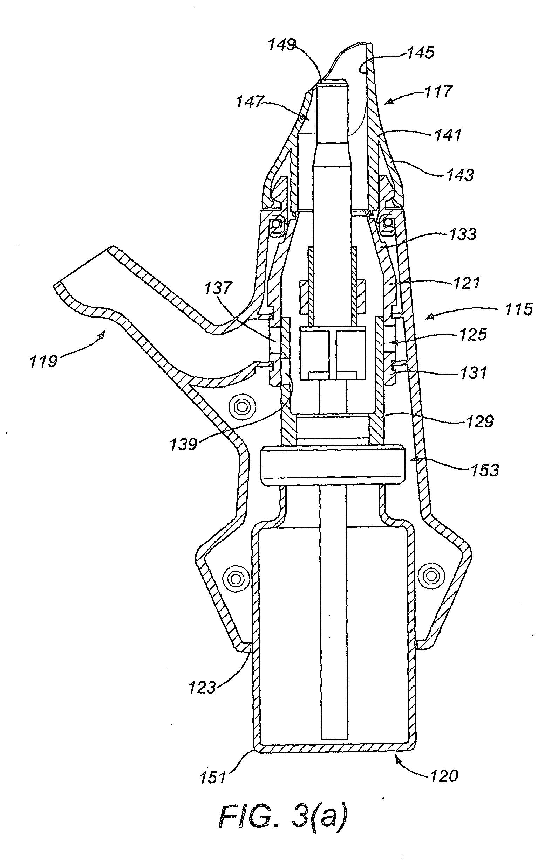

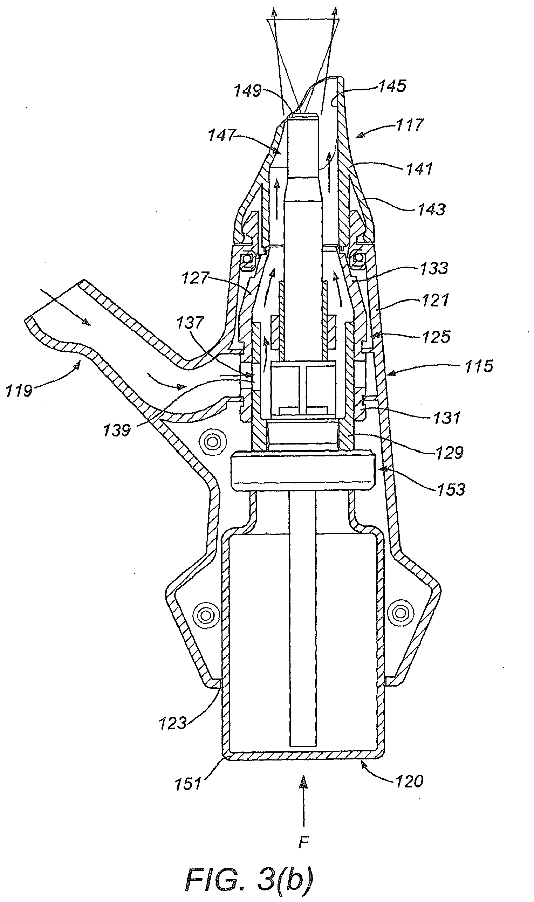

Figures 3(a) and (b) illustrate a Breath Powered^{™} liquid delivery device which is operative to deliver a powder aerosol.

The delivery device comprises a housing 115, a nosepiece 117 for fitting in a nasal cavity of a subject, a mouthpiece 119 into which the subject in use exhales, such as to enable delivery of an air flow into and through the nasal airway of the subject on exhalation by the subject through the mouthpiece 119, and a substance supply unit 120, which is manually actuatable to deliver substance to the nasal cavity of the subject.

The housing 115 comprises a body member 121, in this embodiment of substantially elongate, tubular section, which includes an aperture 123 at one end thereof, through which projects an actuating part of the substance supply unit 120, in this embodiment as defined by the base of a substance-containing chamber 151.

The housing 115 further comprises a valve assembly 125 which is fluidly connected to the nosepiece 117 and the mouthpiece 119, and operable between closed and open configurations, as illustrated in Figures 3(a) and (b), such as to provide for an air flow, in this embodiment in the form of a burst of air, through the nosepiece 117 simultaneously with actuation of the substance supply unit 120, as will be described in more detail hereinbelow.

The valve assembly 125 comprises a main, body element 127 and a valve element 129 which is slideably disposed to the body element 127 between closed and open positions, as illustrated in Figures 3(a) and (b).

The body element 127 comprises a valve section 131, in this embodiment a tubular section, in which the valve element 129 Is slideably disposed, and an inwardly flaring forward section 133, In this embodiment having an Inwardly tapering section, which is downstream of the valve section 131 and fluidly connected to the nosepiece 117.

The valve section 131 of the body element 127 and the valve element 129 each include a valve aperture 137, 139, which are fluidly isolated when the valve element 129 is in the closed position, as illustrated in Figure 2(c), and in fluid communication when the valve element 129 is in the open position, as illustrated in Figure 2(d).

The nosepiece 117 comprises a body member 141 which defines an outer sealing surface 143 for providing a sealing fit between the nosepiece 117 and a nasal cavity of the subject, and an Inner delivery channel 145, which is In selective fluid communication with the mouthpiece 119 such that an air flow is selectively delivered into and through the nasal airway of the subject on exhalation by the subject through the mouthpiece 119, and an outlet unit 147 for delivering substance into the nasal airway of the subject, which is disposed within the delivery channel 145.

In this embodiment the outlet unit 147 comprises a nozzle 149 for delivering substance to the nasal airway of the subject In this embodiment the nozzle 149 is disposed in the delivery channel 145 co-axially with the same.

In a preferred embodiment the distal end of the outlet unit 147 is configured to extend at least about 2 cm, preferably at least about 3 cm, and more preferably from about 2 cm to about 3 cm, into the nasal cavity of the subject.

In this embodiment the substance supply unit 120 is a pump unit, which comprises a substance-containing chamber 151 which contains substance and extends from the aperture 123 in the housing 115 as the actuating part of the substance supply unit 120, and a mechanical delivery pump 153 which is actuatable, here by depression of the substance-containing chamber 151, typically by a finger or thumb of the subject, to deliver a metered dose of substance from the substance-containing chamber 151 to the outlet unit 147 and from the nozzle outlet 149 thereof, here as an aerosol spray.

In this embodiment the substance-containing chamber 151 is coupled to the valve element 129 of the valve assembly 125, such as to be moved therewith and simultaneously provide for actuation of the substance supply unit 120 and opening of the valve assembly 125, whereby substance, here in the form of a spray, and an air flow, here as a burst of air, are simultaneously delivered to the nasal cavity of the subject.

In this embodiment the mechanical delivery pump 153 is a liquid delivery pump for delivering a metered dose of substance, but in an alternative embodiment the mechanical delivery pump 153 could be a powder delivery pump, which delivers metered doses of a powdered substance on actuation thereof.

In this embodiment the substance supply unit 120 is a multi-dose unit for delivering a plurality of metered doses of substance in successive delivery operations.

The present disclosure will now be described herein with reference to the following non-limiting Examples.

### Example #1

The purpose of this study was to study the onset of headache relief following a dose of sumatriptan. The study population included 436 subjects. Study treatments included (i) 16 mg of sumatriptan powder administered intranasafly with the Breath Powered^{™} administration system of the above-described embodiment and (ii) administration of an oral tablet, in which 100 mg sumatriptan was administered orally in conjunction with use of the Breath Powered^{™} administration system but containing no active substance.

Headache relief is defined as a reduction from moderate (grade 2) or severe (grade 3) to none (grade 0) or mild (grade 1) pain. The study compared headache relief at 30 minutes following intranasal administration of a dose of 16 mg of sumatriptan with the oral administration of 100 mg of sumatriptan in the acute treatment of single migraine attack.

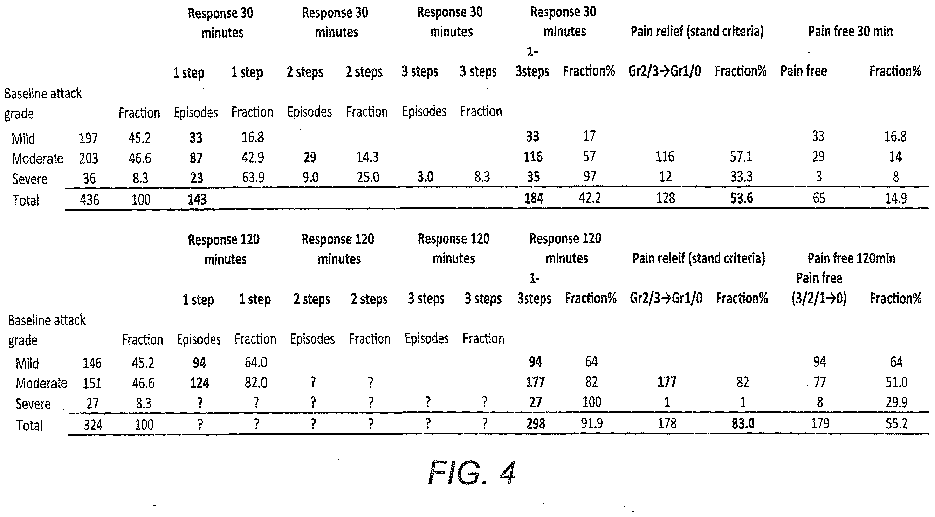

Figure 4 summarizes the response rates in this study at 30 minutes and 120 minutes following administration. As can be seen, the combination of the administration of 100 mg of sumatriptan with the placebo device provided a response rate of 39% at 30 minutes. The combination of the administration of 16 mg of sumatriptan in the Breath Powered^{™} device and oral tablet placebo provides a response rate of 67% at 30 minutes.

A potential mechanism for the earlier onset of action of sumatriptan may be attributed to the fact that carbon dioxide may inhibit the sensory nerve activation and calcitonin gene-related peptide (CGRP) release, and the flow pattern of the carbon dioxide and drug may also play a role. A higher pressure of from 3 to 7 kPa is delivered through the devices of the present Example, which may allow the drug and carbon dioxide to reach the posterior region of the nasal cavity, and in particular target the trigeminal nerve V1. The combination of the carbon dioxide exposure and the mucosal pressure may be advantageous. Carbon dioxide may counteract the NO effect and promote CGRP release. The pH of the nasal mucosa may also change when exposed to a higher pressure and concentration of carbon dioxide.

Other embodiments of the disclosure will be apparent to those skilled in the art from consideration of the specification and practice of the disclosure disclosed herein. It is intended that the specification and Examples be considered as exemplary only, with a true scope and spirit of the disclosure being indicated by the following claims.

### Example #2

This example included a randomized, open-label, single-dose, crossover comparative bioavailability study in healthy subjects, conducted at a single center in the USA. The study population included 20 male and female subjects 18-55 years of age, who were judged healthy by the investigator, with no clinically relevant abnormalities as determined by medical history, physical examination, blood chemistry, hematology (including complete blood count), urinalysis, vital signs, and electrocardiogram (ECG). Eligible subjects had a body mass index (BMI) of 18-32 kg/m2 and a body weight of not less than 50 kg. Prior to inclusion, subjects agreed to abstain from alcohol intake from 48 hours before each administration of study medication and during the period of confinement, and to limit caffeine/methylxanthine intake to less than 300 mg/day for 7 days prior to and for the duration of the study, with no intake from 24 hours before dosing and throughout confinement. Subjects also agreed not to consume food or beverages containing grapefruit, Seville oranges, or quinine (e.g. tonic water) 72 hours prior to study day -1 until after the last pharmacokinetic sample had been collected. and not to consume food containing poppy seeds during the study. Subjects had verified airflow through both nostrils, an ability to close the soft palate (e.g., ability to inflate a balloon) and were able to use the Breath Powered^{™} device of the present Example correctly.

Subjects with a history of migraines, a history of hypersensitivity or allergies to any drug, including sumatriptan or any of its components, or sulphonamides were excluded. Subjects were ineligible if they had a hemoglobin level below the lower limit of normal at screening, had donated blood or experienced significant blood loss (>500 mL) within 3 months prior to screening, or were planning to donate blood within 2 months of completing the study. Use of drug metabolizing enzyme (CYP-450) inducers within 28 days prior to dosing or inhibitors within 14 days prior to dosing, use of any monoamine oxidase inhibitors within 28 days prior to dosing, use of any prescription medications/products, except hormonal contraceptives in female subjects of childbearing potential, and use of any over-the-counter non-prescription preparations (except ibuprofen and acetaminophen used at recommended doses) within 14 days of study entry, all resulted in exclusion. Pregnant and lactating females were excluded. The presence of respiratory diseases or known nasal obstruction, including allergic rhinitis, nasal septum deviation, polyposis, severe mucosal swelling, nasal ulcers, nasal trauma, or for any other reason, a history of chronic nose bleeds, current nasopharyngeal illness, and known vellum insufficiency also resulted in exclusion.

The study consisted of 6 visits. At visit 1, subjects were screened for eligibility. Following a physical examination, subjects were instructed on the use of the Breath Powered^{™} delivery device of the present Example. Once the subject demonstrated an ability to appropriately use the device, the remaining screening procedures (vital signs, ECG recording, blood and urine sampling for clinical laboratory tests, alcohol and drugs of abuse tests, serum pregnancy test [women only]), were performed.

Eligible subjects attended the clinic for 4 additional visits (visits 2-5). At each visit, subjects checked-in to the study site the evening before dosing and remained there until after the last blood sample for determining sumatriptan concentration had been drawn. Randomization was generated by Celerion Bioanalysis Laboratory in Lincoln, NE, USA. Subjects were randomly assigned to treatment sequence using a 4 by 4 Latin square design at the first treatment visit (visit 2). The study treatments were 20 mg sumatriptan powder administered intranasally with the Breath Powered^{™} device; 20 mg sumatriptan nasal spray (Imitrex^{®} Nasal Spray, GlaxoSmithKline); 100 mg oral tablet (Imitrex^{®} Tablet GlaxoSmithKline); and 6 mg subcutaneous injection (Imitrex^{®} Injection GlaxoSmithKline). Each subject received each of the 4 treatments on the 4 separate periods at approximately the same time at each visit, with a 7-day washout between treatments. The subjects fasted for at least 8 hours before dosing and up to 4 hours post-dose.

For dosing, of sumatriptan powder with the Breath Powered^{™} device, subjects first self-administered a 10 mg dose into one nostril and then self-administered a second 10 mg dose into the other nostril. For dosing with the nasal spray, subjects were first instructed on appropriate administration and then subjects self-administered a single dose of 20 mg sumatriptan to one nostril. The oral tablet was taken by subjects with 240 mL water. For the subcutaneous injection, the investigator or designee made the injection of the 6 mg dose of sumatriptan in the subjects' abdomen.

Subjects returned at visit 6 for follow-up evaluations between 3 and 10 days after the last blood draw for sumatriptan concentration determination. Safety evaluations were based on reports of adverse events (AEs), physical examination, clinical laboratory tests, and vital signs and ECG measurements.

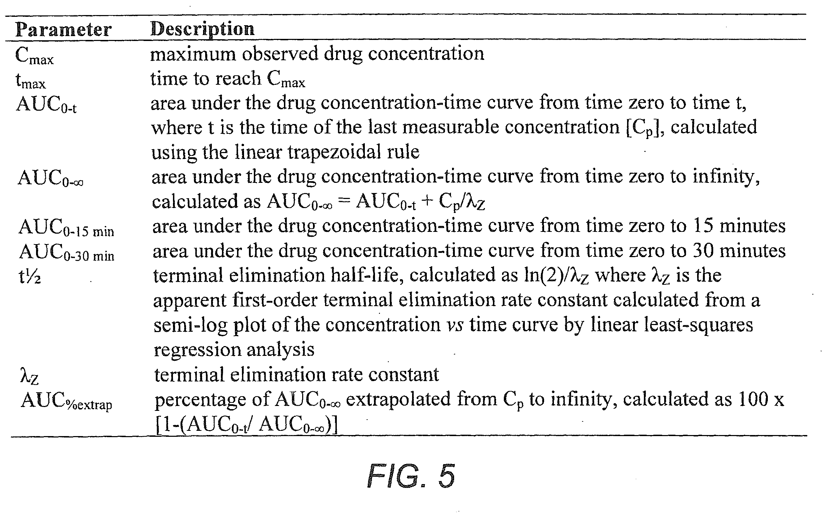

Blood samples (5 mL) were collected in tubes containing K2EDTA at pre-dose (time 0) and 2, 5, 10, 15, 20, 25, 30,45 minutes, 1, 1.5, 2, 3, 4, 6, 8, 10, 12 and 14 hours post-dose. The plasma fraction was separated by placing the collection tube into a refrigerated centrifuge (2 - 8 °C) for 10 minutes at 1,500 x g. All plasma samples were stored frozen at -20 °C until shipped to the bioanalytical facility. Plasma samples were analyzed for sumatriptan at the Celerion Bioanalysis Laboratory in Lincoln, NE, USA using a validated LC-MS/MS method. The lower limit of quantitation (LLOQ) was 0.1 ng/mL, and all concentrations below the LLOQ were treated as 0 for the calculations of descriptive statistics and the PK parameters. All PK parameters were calculated using a noncompartmental approach in WinNonlin Professional^{®} Version 5.2 (Mountain View, CA, USA) and SAS^{®} (Release Version 9.1.3, SAS Institute Inc., Cary, NC, USA). The PK parameters calculated are listed in Figure 5.

The sample size was based on practical considerations rather than statistical power. A sample size of 20 subjects provided at least 5 replications within each sequence using a 4 by 4 Latin square design and was judged to provide a robust evaluation of PK parameters.

The plasma concentrations and PK parameter values were imported into SAS^{®} which was used to calculate all descriptive statistics, An analysis of variance (ANOVA) on the In-transformed PK parameters AUC0-∞, AUCO-t, AUC0-30 min, and Cmax of sumatriptan was used to compare treatments. The ANOVA model included sequence, treatment, and period as fixed effects and subject nested within sequence as a random effect. Sequence effect was tested using subject (sequence) as the error term at a 5% level of significance. Each ANOVA included calculation of least-squares (LS) means, the difference between treatment LS means, the standard error, and 90% confidence intervals (CI) associated with this difference. The LS means, difference between LS means, and 90% Cl of each difference were exponentiated to the original scale. Two treatments are considered bioequivalent only if the 90% Cl of the treatment difference is fully contained within the accepted bounds of 80-125%.

The plasma concentration-time profile of sumatriptan was well characterized for each of the 4 treatments (Figure 6). Overall exposure from both of the intranasally administered sumatriptan treatments was considerably lower than sumatriptan delivered by either the oral or subcutaneous route. The mean plasma concentration-time profiles up to 4 hours post-dose for the two intranasal treatments demonstrate a clearly differentiated profile following delivery by the Breath Powered^{™} device (Figure 7): in the first 30 minutes following dosing, sumatriptan powder from the Breath Powered^{™} device produced a faster rise in plasma sumatriptan concentration and a substantially greater exposure as compared with liquid sumatriptan nasal spray.

A summary of the PK parameters for the 4 treatments is presented in Figure 8. There were no first point tmax values and the mean residual area (defined as AUC%extrap) was approximately 5% or less for all treatments. Intranasal administration of sumatriptan powder using the Breath Powered^{™} device resulted in a 27% higher peak exposure (Cmax), and a 75% higher early exposure (AUC0-15 min) relative to the sumatriptan nasal spray, despite a 20% lower delivered dose. On a dose-adjusted basis, this represents a 59% higher peak exposure and 119% higher early exposure. The extent of systemic exposure as measured by AUC0-t and AUC0-∞ over 14 hours was similar for the Breath Powered^{™} device and the nasal spray liquid sumatriptan. In contrast, the sumatriptan powder delivered with the Breath Powered^{™} device produced a substantially lower peak and overall systemic exposure relative to both the 100 mg oral tablet and the 6 mg subcutaneous injection. Although the absorption profile curve for both intranasal products was characterized by bi-modal peaks consistent with a combination of early nasal absorption followed by late gastrointestinal absorption, these products did not show the same pattern (Figure 7). The early peak was higher with Breath Powered^{™} delivery, while the later peak was higher with nasal spray delivery.

The apparent terminal elimination half-life, at approximately 3 to 4 hours, was comparable following the 2 intranasal treatments and the oral tablet, but was shorter for the subcutaneous injection at approximately 2 hours.

Statistical comparisons of the plasma sumatriptan PK parameters using geometric means are summarized in Figure 9. Although the overall extent of systemic exposure (not dose adjusted) was similar for Breath Powered^{™} delivery of sumatriptan powder and nasal spray, the peak exposure and cumulative exposure in the first 30 minutes post-dose was approximately 20% and 52%, respectively, higher for sumatriptan powder suggesting that more sumatriptan reaches the systemic circulation early after dosing despite the delivery of an approximately 20% lower dose (16 mg vs 20 mg). Relative to both oral tablet and subcutaneous injection, the peak and overall exposure following sumatriptan powder delivered intranasally by the Breath Powered^{™} device was substantially lower.

Quantitative measurement of residuals in used Breath Powered^{™} devices demonstrated that the devices delivered 8 ± 0.9 mg (mean ± SD) of sumatriptan powder in each nostril (total dose 16 mg). Although the extent of systemic exposure over 14 hours was similar following Breath Powered^{™} delivery of 16 mg of sumatriptan powder and 20 mg of liquid nasal spray (AUC0-∞ 64.9 ng*hr/mL vs 61.1 ng*hr/mL), sumatriptan powder, despite a 20% lower dose, produced 27% higher peak exposure (Cmax 20.8 ng/mL vs 16.4 ng/mL) and 61% higher exposure in the first 30 minutes compared to the nasal spray (AUC0-30 min 5.8 ng*hr/mL vs 3.6 ng*hr/mL). The magnitude of difference is larger on a per-milligram basis. The absorption profile following standard nasal spray demonstrated bi-modal peaks, consistent with lower early followed by higher later absorptions. In contrast, the profile following Breath Powered^{™} delivery showed higher early and lower late absorptions.

Relative to the 100 mg oral tablet (Cmax 70.2 ng/mL, AUC0-∞, 308.8 ng*hr/mL) and 6 mg injection (Cmax 111.6 ng/mL, AUC0-∞ 128.2 ng*hr/mL), the peak and overall exposure following Breath Powered^{™} intranasal delivery of sumatriptan powder was substantially lower.

The PK characteristics of sumatriptan powder in the present study show that the initial rate of rise in plasma concentration was faster following Breath Powered^{™} administration of sumatriptan powder than following either the 20 mg sumatriptan nasal spray or the 100 mg oral tablet.

Comparison of various oral and parenteral formulations of sumatriptan indicate that the rate of rise of plasma concentrations during the initial period of absorption gives a good indication of efficacy, and may explain the similar clinical efficacy of a 20 mg conventional nasal spray to that of 100 mg oral tables despite significant differences in plasma levels. It may also explain the efficacy at 60 minutes observed with the Breath Powered^{™} sumatriptan powder device in migraine patients.

Evaluation of the mean absorption profile for the two forms of intranasal administration reveals some key differences. Unlike the range of currently available sumatriptan injection products, which are bioequivalent, PK profiles demonstrate that these intranasal products are not bioequivalent. With the liquid nasal spray, there is a pronounced hybrid absorption pattern with a dual peak, suggesting proportionately lower intranasal absorption followed by a higher degree of what is most likely gastrointestinal absorption, consistent with a large portion of the delivered dose being swallowed. In contrast, the early peak is more pronounced after sumatriptan powder, suggesting a larger proportion of the delivered dose is intranasally absorbed. As presented in Figure 8, differences between Breath Powered^{™} intranasal powder and the standard liquid nasal spray, respectively, are also evident in several metrics characterizing the absorption profiles even before performing dose adjustment for delivered dose, including Cmax (20.8 vs 16.4 ng/mL), AUC0-30 (5.8 ng*hr/mL vs 3.6 ng*hr/mL) and AUC0-15 (2.1 ng*hr/mL vs 1.2 ng*hr/mL). The delay in time to maximum concentration associated with the nasal spray relative to sumatriptan powder (median tₘₐₓ 1.5 hr vs. 0.75 hr, respectively) is also consistent with Breath Powered^{™} delivery producing a higher proportion of early nasal absorption. However, median tₘₐₓ values should be interpreted with caution in the context of bi-modal absorption profiles.

It is worth noting that the sumatriptan powder was administered to two nostrils while the nasal spray was administered to a single nostril. The impact of administering liquid sumatriptan nasal spray in divided doses between both nostrils on the pharmacokinetic profile has been previously investigated and found not to impact either the rate or extent of absorption over administration to a single nostril. Therefore, it is unlikely that this difference in administration procedure explains the findings of the current study.

The dose of sumatriptan powder loaded into the pair of drug capsules delivered using the Breath Powered^{™} device was approximately 20 mg. However, the measured mean delivered dose was 16 mg which is 20% lower than the 20 mg of sumatriptan delivered with the nasal spray. This further accentuates the differences in both the rate and extent of absorption observed between the two different intranasal delivery approaches.

Sumatriptan liquid nasal spray has not been widely used. This may in part be reflective of a lack of motivation due to few significant perceived benefits associated with the nasal spray, which is limited by the Inherent Inadequacies of nasal spray delivery. Given that in many subjects a large portion of drug is absorbed from the gastrointestinal tract, the difference between intranasal delivery and oral delivery may not be observable in many patients. Breath Powered^{™} delivery of sumatriptan powder avoids many of the delivery inadequacies of a typical spray by distributing powder to the area beyond the nasal valve, producing an absorption profile consistent with proportionately more intranasal and less gastrointestinal absorption. The resulting large difference in speed and extent of absorption at the earliest time points after treatment is likely due to a more extensive absorption from the nasal cavity. This study evaluated healthy volunteers; however, a shift towards proportionately greater nasal absorption may be especially important in the clinical context of a migraineur, where the differences between oral dosing and Breath Powered^{™} dosing may be more pronounced than in healthy volunteers. Multiple studies have shown delayed gastric emptying in patients with migraine headache, suggesting risks to reliability and speed of medication absorption after oral dosing and a "rightward shift" of the oral PK curve in such patients. Because rapid rate of rise in sumatriptan blood levels has been hypothesized to produce a faster speed of onset or higher magnitude of treatment efficacy, it is important to note that Breath Powered^{™} delivery was associated with a more rapid initial rate of rise than either oral or nasal spray. Additional theoretical benefits associated with achieving true intranasal deposition augmented by positive pressure exhaled breath include delivery of drug and carbon dioxide to the first branch of the trigeminal nerve and the parasympathetic sphenopalantine ganglion.

Tolerability or safety concerns are sometimes associated with use of injected and oral triptans. This study found there was significantly lower peak and overall systemic exposure following the Breath Powered^{™} sumatriptan powder device compared with either the tablet or the injection. Reduced exposure may translate into a better safety and tolerability profile. This study found Breath Powered^{™} delivery of sumatriptan powder to be safe and well tolerated by healthy subjects, with no systemic adverse events and only a single subject reporting dysguesia. In contrast, 4 subjects experienced flushing following the subcutaneous injection, and 3 subjects each reported nausea following the tablet and the injection.

It is concluded that Breath Powered^{™} intranasal delivery of sumatriptan powder produced a faster and more efficient absorption profile when compared with nasal spray and a substantially lower level of exposure than either the tablet or injection.

### Example #3

Figures 10 to 12 Illustrate sumatriptan PK parameters for nitroglycerin (GTN) induced migraines compared to sumatriptan PK parameters for healthy subjects obtained using the Breath Powered^{™} (OptiNose) delivery device and the Imitrex^{®} nasal spray (GSK).

It is believed that autonomic changes could provide better absorption and effects of unilateral delivery to the side of the migraine. Unilateral activation of the trigeminal nerve could modify the nasal mucosa to offer increased nasal absorption and delayed gastrointestinal absorption. Autonomic activation of the trigeminal nerve could make the administration of carbon dioxide more efficient and the mucosa could become more susceptible to pressure. As can be seen from Figure 10, the 7.5 mg delivered to the side of the migraine during a GNT attack in migraineurs resulted in a bioavailability of 27%. The Cmax for the administration for the side of the migraine is 11, whereas it is only 9.7 for the Imitrex nasal spray. Administration of 7.5 mg to each of the nostrils does not appear to provide a higher bioavailability.

Breath Powered^{™} intranasal delivery of sumatriptan powder is a more efficient form of drug delivery, producing a higher peak and earlier exposure with a lower delivered dose than nasal spray and faster absorption than either nasal spray or oral administration. It also produces a significantly lower peak and total systemic exposure than oral tablet or subcutaneous injection.

### Example #4

This study is a double-blind study with the Breath Powered^{™} device delivering 20 mg of sumatriptan bi-laterally and a 100 mg sumatriptan tablet. The study is a cross-over design where each patient enrolled will treat headaches with each of the treatments. Specifically, patients will treat up to 5 headaches with a treatment and then cross over to treat up to 5 headaches with the other. With each headache, the patient uses the device and takes a tablet, only one of which will be active. From data on over 400 headaches, as yet unblinded, the results obtained at the 30 min timepoint (headache relief 30 minutes after taking medication) for moderate or severe headaches is 54%.

The literature suggests that response at 30 mln from a 100 mg tablet of sumatriptan should be around 9-14%. This indicates that the response rate we are seeing with the placebo device is much higher than what has been previously observed with oral tablets alone.

### Example #5

Intranasal formulations of dihydroergotamine mesylate (DHE), sumatriptan, zolmitriptan, butorphanol, civamide, and lidocaine have all been used/investigated for the treatment of migraine and/or cluster headache. Civamide and lidocaine have been administered via a nasal dropper to interrupt nerve transmission, and although there has been some evidence of clinical efficacy, neither has received US Food and Drug Administration approval for the treatment of headache. Furthermore, nerve stimulation of the SPG has shown promising results in aborting cluster headache, strongly supporting the potential of local treatment to nerves that may be accessed from the nasal cavity.

DHE, sumatriptan, zolmitriptan, and butorphanol have obtained regulatory approval for the treatment migraine and can be administered in the form of a conventional nasal spray by the patient. DHE is known to be a highly effective medication when administered intravenously. Unfortunately, it is less than 1% bioavailable when given orally. However, when administered Intranasally, it has a bioavailability of ~40% allowing for use of this medication in the outpatient setting, In addition to the intranasal formulations, sumatriptan is available as a subcutaneous injection, an oral tablet, suppositories, and a rapid dissolving tablet (outside the United States). In addition to the intranasal formulation, zolmitriptan is available as an oral tablet and fast melt formulation. For both drugs, the intranasal formulations were introduced as alternatives to the oral formulations to overcome the issues of slow onset, reduced GI absorption during headache from slowed motility, as well as the aversion of patients to take oral medications in the presence of nausea.

Both intranasal sumatriptan and intranasal zolmitriptan have demonstrated superiority against placebo in providing relief of migraine symptoms, and intranasal zolmitriptan has been demonstrated to provide earlier relief than the same dose of zolmitriptan oral tablets. Each provides a more rapid absorption than the respective orally administered tablet. However, neither has resulted in a marked increase in total bioavailability relative to oral.

These triptan conventional nasal sprays display a bimodal absorption pattern with a fairly small early peak attributed predominantly to absorption across the nasal mucosa, followed by a later more distinct peak representing GI absorption of the significant amount of drug swallowed after bypassing the nose. For zolmitriptan, the nasal fraction has been quantified in a study and found to account for approximately 30% of the total absorption. A similar study has not been conducted with sumatriptan nasal spray, though sumatriptan liquid nasal spray pharmacokinetics have been studied. It is important to note that the approved dose of zolmitriptan delivered nasally Is the same as the highest dose for tablets (5 mg), whereas the range of approved conventional sumatriptan nasal spray doses (5, 10, and 20 mg) is fivefold lower than the oral doses (25, 50, and 100 mg). Consequently, the systemic exposure is significantly lower for the range of sumatriptan nasal spray doses compared with the oral formulation, whereas it is similar or even slightly higher with nasal zolmitriptan. The opportunity to deliver a lower dose highlights a potential advantage of delivering sumatriptan nasally (vs zolmitriptan) as the risk for systemic and GI-related side effects relative to the oral formulation may be reduced by lowering the systemic exposure.

Despite the theoretical advantages of intranasal drug administration, there have been impediments to broad adoption for the treatment of migraine headache. For patients, the consequences of the inadequate deposition to the target mucosa achieved with traditional nasal sprays is likely a key factor contributing to a lack of perceived clinical benefits over oral treatment. Prospective studies have demonstrated that a key driver for patients preferring a nasal spray is speed of onset In addition, for obvious reasons, alternative formulations that offer the potential of faster absorption may be preferable over simply increasing the dose of an oral formulation. Enhanced tolerability or safety relative to oral formulations would simply add to patient preference should they accompany a core efficacy benefit like improved speed of onset.

Traditional spray pumps used with nasal sprays result in limited drug deposition to the target sites beyond the narrow triangular-shaped constriction called the nasal valve, which is located approximately 2 cm from the entrance of the nostril. The purpose of the narrow nasal valve, in concert with the complex convoluted nasal passageways, is to filter and condition the inspired air, enhance olfaction, and optimize gas exchange and fluid retention during exhalation. These important functional features of the nose impose important limitations on efficient nasal drug delivery that are too often ignored.

For example, the expanding convex spray plume and high particle speed emitted from a spray bottle will largely impact on the walls of the nasal vestibule. Increasing the propulsive force of the nasal delivery does not alter the fundamental anatomic constraints, as the plume impacts on the first surfaces it reaches, while "sniffing" exacerbates the problem as described later. The anterior segment of the nasal cavity, the nasal vestibule, is lined primarily with nonciliated squamous epithelium, which is less efficient for medication absorption than the ciliated respiratory epithelium beyond the nasal valve. Because of this mismatch between the geometry of the anterior region of the nose and the spray plume, only a small fraction of the spray penetrates beyond the nasal valve, and a large portion of the spray volume remains in the vestibule.

The large volume of liquid in the vestibule of the nose may drip out or be wiped off. Sniffing during delivery further narrows the nasal valve, and reflexive sniffing after delivery to avoid drip-out will not only further narrow the nasal valve, which is already particularly narrow superiorly, but also shrink the already slit-like deeper nasal passages. This tends to impair both the intended targeting to a broad nasal surface area and any potential benefits of higher deposition, and tends to direct whatever medication penetrates the nasal valve along the nasal floor to be swallowed, Taste buds sensing bitter taste located at the base of the tongue are quickly exposed to the concentrated liquid that contributes to the intense bitter taste often reported with these nasal sprays, It is only the smaller proportion of the spray that reaches the highly vascularized respiratory mucosa that accounts for most of the early nasal absorption. Such a significant portion of the medication delivered by conventional nasal sprays is swallowed, rather than being nasally absorbed, which the GI tract contributes more to the amount of drug absorbed than does the nose. This phenomenon Is observed with sumatriptan where a bimodal absorption profile is produced following conventional nasal spray administration: a lower early peak, likely related to intranasal absorption, is produced after 20 minutes and is followed by a higher absorption peak consistent with GI absorption around 90 minutes.

The predominance of oral absorption following conventional nasal spray delivery reduces the intended advantages of nasal delivery. Thus, the lack of significant differentiation from oral tablets results in only marginally faster onset of action in some patients and likely contributes to the limited uptake in the market place observed with nasal sprays.

Notably, both the sensory and parasympathetic branches of the trigeminal nerve involved in the pathophysiology of migraine and other headaches innervate the mucosal surfaces beyond the nasal valve, which is also where the SPG resides. To the extent that these structures are involved in headache pathophysiology, the posterior and superior portion of the nasal cavity would be an interesting target for therapeutic intervention with current or future drugs; however, they cannot be effectively reached with a standard nasal spray.

A comprehensive review on deposition patterns associated with nasal drops and spray pumps concluded that traditional delivery devices are suboptimal for delivery to the respiratory mucosa beyond the nasal valve. Several approaches attempting to improve the drug delivery of traditional spray pumps have been suggested and tested over the years, but are generally either impractical, suboptimal, or have yet to be proven in replicated human intranasal deposition studies. Efforts to optimize conventional nasal sprays by improving the method of use have been similarly unrewarding: a study tested 7 different head and body positions using traditional nasal sprays and concluded that there is "no best method."

The Breath Powered Bi-Directional delivery mechanism described herein can be implemented in simple devices without electromechanical cost or complexity, and overcomes many deficiencies of traditional nasal delivery. Both liquid and powder drugs can be delivered using such devices. This nasal delivery concept consists of devices with a flexible mouthpiece and a shaped, sealing nosepiece. It is designed to exploit unique aspects of the nasal anatomy and physiology to improve the extent and reproducibility of drug delivery to target sites in the nose beyond the nasal valve while avoiding the risk of lung inhalation.

In one operation, the user slides the shaped nosepiece into one nostril to create a seal with the nasal tissue, inserts the mouthpiece between the open lips, takes a deep breath, closes the lips around the mouthpiece, and then exhales forcefully into the mouthpiece. The oral exhalation Into the device creates a positive pressure in the oropharynx, naturally elevating and sealing the soft palate and completely separating the nasal and oral cavities. Because of the sealing nosepiece, the airflow and dynamic positive pressure is transferred by the device into the nasal cavity where it expands the nasal valve and narrow slit-like passages. The intranasal pressure, which is slightly reduced compared with the intraoral driving pressure due to the resistance of the device and the nasal passage, balances the pressure across the soft palate to generally avoid over elevation of the soft palate. This generally maintains patency of the communication pathway between the two nostrils that is located deep in the nasal cavity posterior to the nasal septum, permitting the exhaled breath to escape from the contralateral nostril while relieving the nasal cavity of excess pressure.

A dedicated multiuse Breath Powered powder device with a reusable device body and a disposable nosepiece was developed for use in patients with migraine headache. An 11-mg dose of sumatriptan powder is filled into a standard respiratory capsule and provided to the patient in a capsule chamber of a disposable nosepiece. There can be a small entrance for airflow at the bottom of the chamber and a larger opening at the top. Prior to use of the device, a fresh nosepiece can be snapped into the top of the device, and the capsule may be pierced by depressing a button on the device body. Upon exhalation into the device, the pierced capsule can vibrate and/or rotate with the exhaled breath, releasing the powder into the airflow. Drug particles are carried posteriorly by the expanding flow of physiologically warmed air into one nostril, beyond the nasal valve, and can be deposited broadly throughout the deep nasal cavity before the air reverses course and escapes anteriorly through the other nostril (Bi-directional delivery).

Multiple studies evaluating anthropometric differences between individuals were conducted in order to develop the appropriate design of the device in order to accommodate differences In individual nostril size and distances and angles between the mouth and nose. The current design has been found in usability testing as well as clinical trials to be well accepted in terms of comfort and ease of use.

The scintigraphic techniques used in the last decades to study in vivo nasal deposition of liquid and powder formulations are relatively crude and did not allow for reliable absolute or relative quantification of regional nasal deposition and clearance patterns. An improved system allowing reliable quantification of the regional nasal deposition of radiolabeled particles in human subjects has been introduced and used in clinical deposition trials comparing conventional nasal spray devices to Breath Powered devices for both liquid and powder drugs.

In the most recent study, Tc99m-labeled lactose powder was delivered with the Breath Powered powder device. A capsule fill and particle size profile similar to sumatriptan powder was used. For measuring differences in deposition, the nose was divided into 3 horizontal segments, and a vertical dividing line was positioned at the head of the inferior turbinate, and radiation counts within each segment were quantified after administration.

The Breath Powered powder device demonstrated a broader deposition on the regions where nasal mucosa is lined by ciliated respiratory epithelium (especially upper and middle posterior regions, but also the upper anterior and middle anterior regions) with less deposition in the non-ciliated nasal vestibule and significantly greater initial deposition to the upper posterior regions beyond the nasal valve compared with the conventional spray delivery (~54% vs 16%) (Fig. 11a). In contrast, liquid sprays deposited most of the dose (~60% vs ~17%) in limited regions in the lower parts of the nose (Fig. 11a, b).

The regional analyses of deposition and clearance clearly demonstrate that the Breath Powered powder device provides broader exposure to the highly vascularized respiratory mucosa beyond the nasal valve, and particularly improves delivery to the middle and upper regions of the nasal cavity. This should reasonably be expected to translate into more rapid and more extensive drug absorption of suitable medications than is achieved with standard nasal spray delivery. This difference should be possible to measure objectively, as it should be reflected in improved PK and ultimately In improved efficacy. Such studies have now been performed assessing the consequences of delivering sumatriptan in this fashion.

Two studies have evaluated the PK of Sumatriptan delivered with the Breath Powered device. One was a crossover study in 12 migraine patients pretreated with either subcutaneous (SC) injection sumatriptan, or sumatriptan powder delivered with a Breath Powered device, prior to a challenge with nitroglycerine known to induce migraine (GTN-challenge).40 The larger second study was a 4-way crossover study in healthy volunteers comparing sumatriptan powder delivered with a Breath Powered device (15 mg delivered dose split between nostrils) to 20 mg sumatriptan nasal spray (1 nostril), 100 mg sumatriptan tablet, and 6 mg sumatriptan SC injection. In both studies, there was a bimodal absorption pattern representing an initial nasal absorption followed by a GI absorption with Breath Powered delivery (Fig. 12). The initial peak observed in both studies was more pronounced than the peak observed with the standard nasal spray (as measured in the second study), indicative along with other PK parameters of a more efficient and faster systemic absorption with the Breath Powered device (Fig. 12). Absorption also occurred earlier than with tablet delivery but with a significantly lower peak and total systemic exposure than either the oral tablet or subcutaneous injection.

The nasal peak for sumatriptan powder is very similar in the two PK studies, one in migraineurs and one in healthy volunteers, occurring early in both populations. However, the later peak, assumed to represent predominantly GI absorption, is substantially smaller in the study performed in migraineurs during GTN-challenge (Fig. 12). This likely reflects the delayed and decreased GI absorption because of autonomic dysfunction observed in migraineurs that is further accentuated during an attack.

It should be noted that sumatriptan powder was split between the two nostrils while the nasal spray was administered to a single nostril. The impact on the PK profile of dividing the liquid spray dose between nostrils has been previously investigated and found not to improve either the rate or extent of absorption over administration to a single nostril. Therefore, it seems unlikely that this difference in administration procedure explains the findings in the PK study in healthy subjects.

It Is important to recall when reviewing the pharmacokinetic data that the total delivered Sumatriptan dose with the Breath Powered delivery device is 20-25% lower than the sumatriptan 20 mg liquid spray. A shift to greater nasal absorption with Breath Powered delivery reduces the fraction of Sumatriptan bypassing the nose compared with sumatriptan spray, and the dose is split between the two nostrils (Fig. 12). The lower delivered dose, broader nasal distribution, and significantly altered clearance pattern (note, the soft palate is usually substantially closed at the time of delivery) following Breath Powered delivery further reduce the amount and concentration of drug reaching the taste buds at the base of the tongue, which is likely to mitigate the intensity of the bitter taste sensation. The results show that the enhanced nasal deposition produced by the Breath Powered device is indeed associated with pharmacokinetic advantages.

It is reasonable to hypothesize that the increased early absorption may offer advantages in terms of improved efficacy and in particular more rapid onset of pain relief, and that the low dose may enhance tolerability or safety. The ability to prevent migraine attacks in the study with GTN-challenge combined with the similar electroencephalography findings following SC and Breath Powered powder delivery, despite much lower blood levels, also suggest potential clinically relevant advantages. These findings provided the rationale to proceed to a randomized placebo-controlled trial with a Breath Powered sumatriptan delivery device.

In the first placebo-controlled, parallel group, 3-arm trial in acute migraine (117 total patients), two doses of sumatriptan powder were delivered with the Breath Powered device and compared with a "placebo" control group using dummy devices. Fast onset of pain relief was observed for both active doses with early pain relief rates similar to historical data for SC injection despite much lower systemic exposure. Significant benefits were also observed for pain relief at 120 minutes for both doses, and the higher dose was selected for further development. The higher dose produced a response of 80% vs 44% with placebo (P < .01) at 2 hours, and high early response rates at 60 minutes (74% vs 38%, P < .01) and at 30 minutes (54% vs 31%; NS).

A phase III, placebo-controlled, parallel group, 2-arm study in 212 patients was recently conducted with sumatriptan powder being delivered with the Breath Powered device. As discussed and shown below, at 2 hours post-dose, a significant proportion of patients experienced pain relief compared with placebo (68% vs 45%, P < .01), a high value for triptan therapy, However, again, the most striking result was the fast onset of pain relief, with a remarkably high response rate at 30 minutes (42% vs 27%, P < .05). This is particularly notable in light of the extremely low dose of a triptan medication. The reported adverse events were primarily mild and transient and generally limited to the site of administration. It was concluded that Breath Powered delivery of intranasal sumatriptan powder is effective, safe, and well tolerated and can offer fast onset of pain relief in adults with acute migraine headache.

### Example 5(a)

The objective of the study was to compare the efficacy and safety of Breath-Powered sumatriptan powder to placebo in the treatment of patients with moderate to severe migraine headache. Patients taking oral triptans commonly cite slow onset of action, inadequate pain relief, and adverse effects as reasons for dissatisfaction; nausea or vomiting can also be a barrier to use. Adverse effects known as "triptan effects" are most often associated with formulations and doses that produce higher plasma levels. In a small trial, low dose sumatriptan powder delivered with an Breath Powered device produced a headache relief rate approaching that previously reported with injections without the attendant side effects. These results supported conduct of a larger trial.

Single-dose, multicenter, randomized, double-blind, placebo-controlled, parallel-group study. Patients had history of migraine for >1 yr prior to entry and reported >1 headache, but <15 headache days, per month. Patients were randomized to a Breath Powered device containing either 20 mg of Sumatriptan powder (15 mg emitted dose) or placebo. Patients treated an attack reaching moderate or severe intensity and recorded symptoms at scheduled times.

The results are shown generally in Fig. 13. Specifically, 223 patients received treatment (112 sumatriptan powder and 111 placebo). The mean age was 42 yrs.; 85% were women. For the primary outcome, 68% of patients in the sumatriptan powder group reported pain relief at 120 min vs. 45% in the placebo group (p<.01). Pain relief curves diverged early, reaching statistical significance at 30 min (42% vs. 27%; p<.05). At 120 min, 37% of patients receiving sumatriptan powder had reported complete relief compared with 17% for placebo (p<.01), while 70% vs. 45% reported meaningful relief (p<.001). Among patients with pain relief at 120 min, 65% sumatriptan powder and 53% placebo (ns) had continued pain relief at 24 hrs. Large reductions in nausea, phonophobia, and photophobia were reported In both groups; between-group differences were not statistically significant. No systemic adverse events were reported in more than one patient. Only one patient reported mild and transient tingling in the hands and head. The most common (>5%) AEs reported were product taste (22%), nasal discomfort (13%), and rhinitis (6%); all transient and generally mild.

This study replicates the previous finding that the Breath Powered device delivering low dose sumatriptan powder produces early headache relief in a high percentage of patients compared to placebo and to historical rates with oral treatment, and a high rate of headache relief. Treatment was well tolerated, with few systemic adverse effects.

Comparison of these results with published data suggest that the speed of onset of pain relief is much faster than oral treatment and approaches that achieved with SC injection, but with substantially lower systemic exposure and therefore the attendant risk of adverse events.

In each clinical trial with Breath Powered delivery, an interestingly high placebo response rate has been observed. In these trials, control patients did not receive "no treatment" but used identical Breath Powered delivery devices to active patients. Although the high response among these "placebo" patients may be due to chance, secular trends, or other factors, it is interesting to note that there are also potential explanations directly relating to the use of the Breath Powered device.

During normal respiration, there is minimal exchange of air in the upper narrow part of the nose. The particular aerodynamics of the Breath Powered delivery device blowing a large amount of exhaled air with about 5-6% carbon dioxide at a flow rate of about 30 L/minute or more lasting for about 2 to about 3 seconds, which penetrates the upper narrow segments of the nose, could provide therapeutic effects, in part similar to those reported with the delivery of 100% carbon dioxide, albeit that this carbon dioxide delivery was done for short duration and done at low flow (10 mL/s) and low volume. In the present Breath Powered device, it is postulated that the oscillating capsule and airflow may significantly enhance exchange of air in upper narrow parts of the nose, as in part observed in response to humming and pulsating nebulizers. In addition, there are reasons to hypothesize that potential positive effects mediated by the positive air pressure, rapid vibrations produced by the rattling capsule, and the removal of NO may all play a role in alleviating migraine headache. One or more of these, or other, device-related mechanisms may contribute to the high response rate In the placebo groups in the trials with Breath Powered powder delivery in migraine patients.

The deep nasal cavity deposition associated with Breath Powered delivery enables the potential for medications to be delivered more broadly to the trigeminal nerve innervated tissue and to the SPG, which may prove to be beneficial in the treatment of a range of headache disorders, The aerodynamic properties of the device itself may offer alternative mechanisms of action and/or synergetic effects.

In addition to possibilities in preemption or prevention of migraine, cluster headache and trigeminal neuralgia represent target indications for possible delivery of numerous new or current drugs alone or in combination, including for example triptans, DHE, lidocaine, nonsteroidal anti-inflammatory drugs (NSAIDs), locally acting corticosteroids, and potentially CGRP-antagonists. There is great unmet need, and it is possible to modify the current device to optimize delivery for treatments intended to particularly target the region closest to the SPG for optimal efficacy. Other potential indications Include chronic migraine, where delivery of a very small daily dose of a triptan or other drugs in this manner may offer sufficient receptor blockage to reduce the number of acute attacks. Even topical steroids may prove valuable alone or as an adjuvant therapy in cluster headache or in sinus headache.

Nasal drug delivery has long been a route of administration known to be useful in the treatment of headache and other disorders. However, the typical methods of intranasal delivery are relatively ineffective in their delivery of medication broadly and to the posterior/superior areas of the nasal cavity where rapid and efficient drug absorption and other benefits can effectively accrue. Therefore, the promise of intranasal drug delivery has not been fully realized. Human gamma-deposition studies in vivo with Breath Powered devices have proven that this novel device mechanism is capable of producing a significantly improved nasal drug deposition pattern. Pharmacokinetic studies to assess the consequences of this improved deposition were performed following the delivery of a low dose of sumatriptan powder, and show that this improved delivery is associated with enhanced speed and efficiency of absorption across the nasal mucosa with a reduced proportion of GI absorption relative to standard nasal spray. In replicated clinical trials, Breath Powered delivery of low dose sumatriptan has now been shown to produce substantial response rates, with early pain relief more similar to SC injection than to other forms of delivery, but with much lower exposure than with oral or SC treatment. This new form of nasal delivery may offer a number of interesting therapeutic options for the treatment of a range of headache disorders in the future.

### Example #6

In another example, it was found that a 20-mg nominal dose of sumatriptan dry powder using the Breath Powered^{™} delivery device (BPPSIT) delivers 16 mg in the nose. This means that the total exposure to sumatriptan with the device is a lower total milligram dose than tablet, nasal spray, or injection. However, directly comparative pharmacokinetic studies show that the 16-mg BPPSIT powder treatment produces higher peak concentration (Cmax ng/mL) than the 20-mg conventional liquid sumatriptan nasal spray (20.8 mg vs 16.4 mg, unadjusted for dose). Both intranasal formulations produce a substantially lower peak concentration (Cmax ng/mL) than either the sumatriptan tablet (100 mg tablet = 70.2, 6 mg) or the subcutaneous injection (6 mg = 111.6 mg). Similarly, total drug exposure as measured by area under the curve (AUC0 ng*hr/mL) is much lower with the intranasal formulations (BPPSIT = 64.9 mg, conventional sumatriptan liquid nasal spray = 61.1 mg, unadjusted for dose) than with the 100 mg tablet (308.8 mg) or injection (128.2 mg). The sumatriptan powder delivered with the BPPSIT is not bioequivalent to any tested Sumatriptan product. Of particular note, the pharmacokinetics of the BPPSIT show a pattern of faster and more efficient absorption than the conventional liquid nasal spray, yielding >60% higher early plasma exposure with an AUC0-15 minutes of 2.1 for BPPSIT vs 1.2 for liquid sumatriptan nasal spray and an AUC0-30 minutes of 5.8 for BPPSIT vs 3.6 for the conventional spray despite the delivery of 20% less drug.

The Phase 2 randomized controlled trial on BPPSIT published in 2010 included 117 adult subjects with episodic migraine. There were 3 arms, a Sumatriptan powder 10 mg arm, a sumatriptan powder 20 mg arm, and placebo. All treatment groups, including placebo, used breath-powered bidirectional devices. As in the Phase 3 trial discussed later, subjects were instructed to treat when migraine was moderate or severe. The Phase 3 trial used only the 20-mg nominal dose, which as noted delivers 16 mg in the nose, so only those data are reviewed.

In the Phase 2 trial, 2-hour pain freedom occurred in 57% of the 20 mg subjects and 25% of the placebo subjects (P <.05). Two-hour headache relief, defined as headache moving from moderate to severe down to zero or mild, was quite high and statistically significant at 80% for 20 mg, and 44% for placebo. Both doses statistically separated from placebo for headache relief by 60 minutes. The most frequent treatment-related adverse event was a metallic taste, occurring in 13% of the 20 mg subjects.

In the Phase 3 regulatory pivotal study on the BPPSIT 20 mg, the TARGET study, there were 223 subjects randomized who received treatment (112 BPPSIT and 111 device loaded with placebo). The primary outcome measure was 2-hour headache relief, which occurred in 67.6% of subjects in the BPPSIT group vs 45.2% in the placebo group (P <.01). For headache relief, BPPSIT reached statistically significant separation from placebo earlier than in the Phase 2 trial, this time at 30 minutes (41.7% vs 26.9%; P <.05). Pain freedom at 2 hours occurred with 34% of BPPSIT subjects compared with 17% for placebo (P <.01).

Adverse events occurring >5% included abnormal taste (22%), nasal discomfort (13%), and rhinitis (6%). No serious adverse events occurred in the pivotal trial.

There are a number of issues worth exploring with the BPPSIT data. These include the difference in efficacy between the Phase 2 and Phase 3 studies, overall efficacy, early response, and the placebo response and therapeutic gain (TG). The data from Phase 2 were dramatic with about an 80% headache relief mark at 2 hours, but in Phase 3, the 2 hour number was not as high, coming in closer to the high end of the conventional triptan range at around 67%, with the 30 minute number at 42%, notably higher than has been reported with oral treatment and in the range of injectable triptans. This can probably be accounted for simply by the number of subjects, with more than double the number in Phase 3 than Phase 2. There are numerous instances of clinicians revising their evaluation of a medication from Phase 2 to 3 because of differences in outcomes becoming apparent with a greater number of subjects (N). With smaller numbers of subjects, results are more at the mercy of random variation.

However, it is possible that the response rate is indeed higher with BPPSIT, and one possibility is that the device is the reason. That is, perhaps a higher response accrues when sumatriptan is delivered high up in the nose, close to the lateral margins which abut the pterygopalatine canal containing the sphenopalatine ganglion and the maxillary division of the trigeminal nerve. The possibility of a direct triptan effect on these pivotal structures for migraine and cluster might merit further exploration.

Although headache relief at 2 hours has been the standard primary outcome variable for most Phase 3 migraine trials, because it is a single time point it does not provide information on the early effects that are considered by patients to be clinically important For BPPSIT, the response at 30 minutes ranged between 42% and 49%. This is a high rate of response for this early time point. Data from randomized controlled regulatory trials included in the Food and Drug Administration-approved prescribing information for nearly all approved triptans provide graphics of pooled efficacy data describing headache response. Review of these graphics reveals that for Sumatriptan injection the headache response at 30 minutes is in the range of 50%, while 30 minutes pain relief is 10-20% for oral formulations, and between 20 and 30% for conventional nasal spray formulations. These data suggest that BPPSIT early response rates may be closer to those observed with injection than has been reported with other non-parenteral delivery forms.

It is interesting that such a low actual dose of 16 mg could have efficacy approaching injection early on, and comparable efficacy at 2 hours to tablets of 6 times the dose. Generally, exposure to lower doses with comparable efficacy is attractive when contemplating the potential for adverse events.

Further inspecting the BPPSIT Phase 3 trial, the placebo rate seems quite high, at 45.2% for 2-hour headache relief; it was also high at 44% in the Phase 2 trial. In contrast, in Ryan and colleagues' paper summarizing the 2 Phase 3 trials for the conventional sumatriptan liquid nasal spray, the placebo rates for 2-hours headache relief were 29 and 35%. There has been a trend for placebo rates to creep up over time in triptan randomized controlled trials. For example, in the trial used to approve sumatriptan oral tablets, the placebo response rate was 17%. There have been numerous hypotheses to explain the rising placebo response rate, including the absence of triptan naïve patients with accompanying rising patient expectations for triptans, and changing study populations as the background pool of patients is influenced by wide availability of triptans.

In the case of BPPSIT, the device itself may be a cause for the high placebo response rate. Many investigators have noted higher placebo rates in the setting of device trials. As one set of investigators noted, "The placebo/nocebo response to sham therapy with a device is similar to that previously reported for prolonged drug treatment" One possibility for the high placebo response rate in the Phase 3 trial was the novelty and use of the device itself.

A technical reason for the high placebo response may be that this Phase 3 trial had a notably low proportion of severe headaches at baseline at 17%, where previous triptan studies typically have shown a higher proportion of severe headaches. Fewer severe relative to moderate baseline scores would be expected to result in higher placebo response given standard scoring scale and analysis methods.

Is it possible that the placebo arm was providing active treatment? The placebo for the BPPSIT trials was treatment with the OPTINOSE device (pressure with carbon dioxide and lactose powder). While one would think that this was a clear sham treatment, in fact there is a literature on the beneficial effects of carbon dioxide on migraine. Spierings and colleagues found in a preliminary trial available only in abstract form that continuous carbon dioxide infusion for acute treatment of episodic migraine resulted in 2-hour pain free responses that were highly statistically significant compared with placebo (25.0% vs 4.8%) (P = .006).

It turns out that carbon dioxide is probably part of the pain regulatory system. Vause and colleagues wrote about their findings in cultured rat trigeminal ganglion cells in 2007, "Incubation of primary trigeminal ganglia cultures at pH 6.0 or 5.5 was shown to significantly stimulate calcitonin gene-related peptide(CGRP)release ... carbon dioxide treatment of cultures under isohydric conditions ... significantly repressed the stimulatory effects of KCI, capsaicin, and nitric oxide on CGRP secretion. carbon dioxide treatment under isohydric conditions resulted in a decrease in ... capsaicin-mediated increases in intracellular calcium [providing] the first evidence of a unique regulatory mechanism by which carbon dioxide inhibits sensory nerve activation, and subsequent neuropeptide release. Furthermore, the observed inhibitory effect of carbon dioxide on CGRP secretion likely involves modulation of calcium channel activity and changes in intracellular pH."

Thus, it is possible the carbon dioxide "sham" of the BPPSIT may have been delivering partial treatment and is thus not a real placebo response. The fact that both Phase 2 and Phase 3 studies showed high placebo response rates of 44-45% suggest this possibility. However, there is precedent for high placebo rates in novel triptan delivery trials. In the first rizatriptan orally dissolvable tablet trial, the placebo rate was 47%. We do not know the concentrations of carbon dioxide in the Spierings device to compare with the BPPSIT, and this further limits our opportunity currently to explore this possibility.

Another issue to consider with the BPPSIT Phase 3 data is that of TG, defined as the difference obtained when placebo response is subtracted from active response. The TG in Phase 2 for 2-hour headache relief for 20 mg was 36; in Phase 3, it was 22. This second TG at first seems to be on the low end for a triptan. If one were to choose to use TG across studies (and more on that later), in fact, the 2 BPPSIT TGs would appear comparable to those for sumatriptan liquid nasal spray. The TGs in the 5 trials of conventional Sumatriptan liquid nasal spray were 25, 25, 29, 35, and 36.

Sheftell and colleagues evaluated whether transformation of triptan efficacy data into TG is useful. The intent of TG is to tease out the true drug effect in the face of placebo variation. To our surprise, it turned out that TG correlated more strongly with placebo response than active response. We stated that TG should not be used to compare triptans, and cautioned that migraine therapies can only be compared using well-designed head-to-head studies and not by meta-analysis.

For analysis purposes, this issue was revisited and compared 2-hour headache relief reported in package inserts by study for active and placebo responses (see Fig. 14, 15). The theory of TG is that the active to placebo response rates should be positively correlated, better than an active-to-active correlation. The response observed with active treatment must rise and fall commensurately with the observed placebo response rate in order for TG to be a useful concept in interpretation of migraine trials.

However, perhaps unlike other applications of the TG concept, it is clear that placebo response rate is widely variable but has little or no impact on the active response rate. Data across the class of triptans show that there Is large variability In placebo response between studies of a given drug, seen In Figure 15 on the X axis. There is much less variability in the active response rate for a given active treatment between studies, seen as a relatively flat line on the Y axis in Figure 15 across the placebo rates. There is no observable correlation between the response observed in placebo and active groups. For the studies pulled, the active:placebo R2 = 0.02.

Active response rates are a superior reflection of true treatment effect than TG, which appears to not be a useful concept In migraine, but as stated in 2001, well-designed head-to-head studies remain the standard for comparison. As noted earlier, it may be fair to say that the headache relief rates for the BPPSIT appear in line with other triptan therapy historically at 2 hours, and possibly approaching historically reported response rates with injectable. Sumatriptan at 30 minutes. This fast onset may be important to patients, particularly those with a need for rapid onset as discussed earlier. And to repeat, it is notable that this response is achieved with such a low delivered dose at 16 mg. Again, this suggests the potential for desirable safety or tolerability compared with higher dose treatment, but also underscores interesting questions about the possible contributions to efficacy of a unique activity of the device or drug in the nasal cavity.

The acute treatment of migraine requires matching individual patient need to drug and formulation. In particular, nausea and vomiting, quick time to peak intensity, and indeed the common gastroparesis of migraineurs, all call for a variety of non-oral formulations for treatment of attacks. As generic triptans become available, attempts to use them in new formulations progress. A novel BPPSIT offers an improvement, at the very least in pharmacokinetics, over conventional liquid nasal sumatriptan spray.

The device used for drug delivery in this breath powered nasal sumatriptan uses natural nose anatomy to close the soft palate and propel the low dose powder sumatriptan high up In the nasal cavity on one side. This approach may reduce adverse events and improve efficacy.

It is certainly a worthwhile endeavor to create new delivery systems for known effective migraine medications. The clinical role for a fast acting non-oral nasal formulation will be, as noted, in those for whom tablets are bound to fail, that is, in the setting of nausea and vomiting or when the time to central sensitization, allodynia, and disabling migraine is too short for the patient to respond to a tablet, given the unpredictable and slower absorption profile of oral medications. Further studies should elucidate whether this novel system affords the predicted benefits clinically in speed of onset and effectiveness, with reduced adverse events compared with earlier non-oral formulations.

### Example #7

In another study, nasal pH measurements during Breath Powered^{™} Bi-Directional^{™} delivery were analyzed. In some aspects, these data could be considered realistic and accessible methods to verify "device effects" *in vivo.* However, measurements of NO and carbon dioxide levels in the nose are not typically feasible as they require constant suction of air from the nose that would change the flow patterns.

One set of data include blinded head-to-head (H2H) results. They generally show a high response rate in blinded data, i.e., a reduction from severe/moderate migraine or mild or none. In addition, potential scenarios after un-blinding at 30 minutes suggest one or more "device effects."

Assuming that the highest active response rate at 30 minutes of Sumatriptan 100 tablets (13%) is added to the highest placebo rate at 30 minutes for the 16 mg (31%) sums to become 44% at 30 minutes. This data suggest a response rate for 16 mg nasal with Placebo tablet of 70% at 30 minutes, which is very high. For the 174 severe attacks, 95% were improved at 30 minutes. Again this is a very high response rate with both treatment options (minimum 90% response).

For the "blinded" data, there were 1556 attacks. Of these, only response data at 30 mins show: 713 Attacks were mild when treated, 669 attacks were moderate when treated, and 174 attacks were severe when treated. For the mild attacks, 117 (16.4%) went to none at 30 min. For the moderate attacks, 288 (43%) went to mild and 101 (15.1%) went to none. For the severe attacks 77 (44.3%) went to moderate, 65 (37.4%) went to mild, and 22 (12.6%) went to none. For all attacks, the 1 pt improvement was 43% and pain freedom was 15.4%. For moderate/severe attacks (n=843), 57% went to mild/none and 14.6% achieved pain freedom. These results are generally summarized in Fig. 16.

Certain physiological aspects of bi-directional flow patterns were reviewed. Generally such flow patterns provide exhaled carbon dioxide exposure to nasal mucosa ranging from about 5 to about 6% carbon dioxide. In addition, pH may change locally In nasal mucosa (Djupesland 2014). Removal of NO from upper part of the nose (Djupesland 1999) may also occur, and positive pressure may be applied to nasal mucosa (Valsalva and pain relief). Furthermore, vibrating airflow may enhance gas exchange from narrow slit-like passages and sinuses. Humming and other publications describe nasal NO, vibrating mesh, and pulsed nebulizers.

There are several possible explanations for the potential device effects described above. Evidence of such effects comes from high placebo rates observed in Phase 2 and Phase 3 trails even at early time points. The blinded H2H data also suggest "additional device effects."

One hypothesis is that bi-directional delivery of exhaled air with about 5-6% carbon dioxide offer similar exposure of carbon dioxide to the nasal mucosa as low flow delivery of 100% carbon dioxide at very low flow rates (see Capnia data) or 15-45% carbon dioxide at low flows (see Schusterman, 2003). In the Capnia phase 2 migraine trial (Spierings, 2008), carbon dioxide was passively delivered at 10 ml/sec for 90 seconds (900 ml) or 5 x 15 (1050 ml) with 45 seconds pauses and up to 7 dosing cycles during first 2 hours with minimum 3.5 minutes resting for migraine. This was about equal to 10 ml of carbon dioxide per second. Considerable dilution of the carbon dioxide is expected due to open nose and possible nasal inhalations or exhalation during delivery.

The Capnia AR trial (Casale, 2008), passive delivery included administering subjects with gases intranasally twice for 60 seconds at a rate of 10 mL/s, for a total dose of approximately 1200 mL. The doses were separated by an interval of less than 5 minutes and were administered to alternate nostrils. The subjects avoided inhaling the gas by breathing through the mouth, allowing the gas to flow in 1 nostril, pass through the nose and sinus cavities, and pass out through the other nostril. Again, flow rate was 10 ml carbon dioxide per second. Considerable dilution of the carbon dioxide is expected due to open nose and possible nasal inhalation or exhalation during delivery.

The Shusterman 2003 article also describes, synchronized with inhalation, 5 l/min 15% x 3 seconds. This equates to 250 ml x 0.15 = 37.5 ml carbon dioxide, or 12.5 ml per second. By comparison, the Breath Powered Bi-Directional delivery (Djupesland 2014), provides 30 l/min for 3 seconds of 5% carbon dioxide = 500 ml/sec with about 5-6% carbon dioxide = 25-30 ml/second or 75-90 ml in 3 seconds. In summary, carbon dioxide has shown effects in migraine (Capnia - Phase 2) and carbon dioxide has shown effects in allergic rhinitis (Capnia - Phase 2). Also, carbon dioxide is believed to act on trigeminal nerves via reduced local pH in mucosa, triggering intercellular events desensitizing the nerve. And carbon dioxide delivered to nose can cause pH change in nasal mucosa (Shusterman, 2003).

It was determined that it may be possible to detect nasal PH changes with small probe following Bi-Dir procedure. We describe above the potential effects of the aerodynamics, potential about 5-6% carbon dioxide exposure In expired air, removal of NO, and pressure effects.

Carbon dioxide works in migraine (and AR) by changing pH (Capnia, CA). A recent publication from 2013 describes the release of CGRP from the trigeminal sensory fibers upon irritant stimuli (carbon dioxide) inhibits the odor response of olfactory receptor neurons. Papers by Vause and Spierings state that "[r]esults from this study provide the first evidence of a unique regulatory mechanism by which carbon dioxide inhibits sensory nerve activation, and subsequent neuropeptide release. Furthermore, the observed inhibitory effect of carbon dioxide on CGRP secretion likely involves modulation of calcium channel activity and changes in intracellular pH."

It appears that it Is the intracellular pH changes that mediate the effects and that the extracellular PH changes to a large extent are buffered by the nasal mucus secretion. However, a recent study as well as the studies by Shusterman (2003) could reliably detect small changes in the nasal pH measure by probes Inserted Into the nasal passage with a diameter between 1.5 and 2 millimeters. These probes have been used to measure pH in esophagus and ventricle, and can be coupled directly to software that provides detailed curves (see example below). It appears that carbon dioxide concentration >15% are required to see a change in the nasal pH. This would speak against the likelihood of seeing changes with concentration of 5-6% in exhaled air even if it actually reached this level with bi-directional delivery. However, the 15% carbon dioxide was delivered to the nose in a way where it is likely to be substantially diluted. The carbon dioxide probe was placed 4 cm into the nose along the floor of the nose and carbon dioxide was administered in 3 second pulses at a flow rate of 5 L/min via a cannula to the front of the nose and synchronized with inhalation (about 0% carbon dioxide). The cannula placed in one nostril was non-occluding. The inhalation flow may thus be substantially higher that the 5 L/min through one nostril and the 15% carbon dioxide may have been substantially diluted at the site of the mucus around the pH probe. In accordance with the estimates presented above, where the mixing and dilution of the carbon dioxide will be much more extensive and rapid than what might be the case in the olfactory region after Breath Powered Bi-directional delivery. The changes in carbon dioxide and related extracellular pH may of course prove to be too small to be detectable by the pH probe, but the only way to find out is by testing.

In some aspects, monitoring equipment (Medtronic, MN, see attached data sheet), or similar equipment may be used for "look and see" experiments. For example, some probes are reusable versions and others as single use versions. A 1.8 mm probe can be inserted into the olfactory region under endoscopic control and then used to measure pH during periods of not breathing, regular slow breathing, and during Bi-directional delivery of air. In addition, Lactose and Sumatriptan can be co-administered to observe any changes or trends. Such data may explain the "placebo" effects, or document the extent to which the effects are real and not placebo.

Previous literature describes rats having hypersensitive olfactory receptors that can sense or smell carbon dioxide concentrations of the order of 1-3% and even lower. This high-sensitivity mode of carbon dioxide detection depends on the activity of carbonic anhydrase which catalyzes the synthesis of carbonic acid et al. The resulting acidification induces activity in a small subset of olfactory receptor neurons which are located in the most dorsal recesses of the olfactory epithelium.

In humans, there is no such high-sensitive carbon dioxide detection, and carbon dioxide has no odor for us. At higher carbon dioxide concentrations, however, trigeminal fibers are activated, again through acidification. Importantly, the protons that induce trigeminal activity are not those released in the olfactory mucus or in the interstitial fluid, but those released within the axoplasm of the trigeminal fibres. Studies of TRPA1-channel gating in trigeminal ganglion neurons recently revealed that the channels are opened by intracellular acidification (Wang et al., 2010).

As carbon dioxide can readily diffuse across plasma membranes, the carbonic anhydrase reaction inside the sensory endings can trigger a drop in intra-fiber pH. The precise extent of this intracellular acidification has not yet been measured, and the intra-fiber concentration of carbonic anhydrases is not known. However, considering the small accessible volume within the fibers, acidification would be expected to be more pronounced within the fibers than in the surrounding fluid with its much larger volume.

In human subjects, Shusterman (2003) measured the acidification of nasal mucosal pH with extracellular pH electrodes during carbon dioxide stimuli similar to the ones used in the present study (5 L/min, 3 s duration, 20% carbon dioxide). The extracellular pH decreased from basal levels of ∼7.4 by only 0.05-0.1 pH units the effect of carbon dioxide is during each carbon dioxide pulse. These minute decrements in extracellular pH reflect efficient pH buffering of the extracellular medium. The advantage of carbon dioxide detection by intracellular acidification is that larger pH changes can be triggered by carbon dioxide inside the axoplasm. With respect to the extracellular medium, the trigeminal fibers appear not to act as pH electrodes but rather as carbon dioxide electrodes, independent of volume and pH buffer capacity of the surrounding fluid.

Even if humans do not have the high-sensitivity to carbon dioxide, recent study suggests that humans may distinguish carbon dioxide levels of about 5-6% CO. Moreover, the nasal mucosa may be more sensitive in the anterior part of the nose.

One or more factors may affect the response data described above that result from bi-directional delivery. One hypothesis is that by performing bi-directional delivery, the particular airflow and pressure characteristics achieved offer separate advantages which may at least In part explain the high placebo effects we have seen in previous studies and the high response we are likely to see at 30 minutes when placebo is combined with the 100 mg Sumatriptan table. We predict that one or more factors may have an impact and these factors are likely to include pressure, removing NO from the nose, or exposure of about 6% exhaled carbon dioxide. Of these factors, the carbon dioxide may have the most significant impact

Carbon dioxide is known to have an effect on migraine and in allergic rhinitis. It is likely that is mediated through small changes in the local pH. A prior study shows that exposure of 5 L/min carbon dioxide in concentrations of 15% and 45% both create dips in mucosal pH of 0.1-0.2 pH units. The study speculated that such small pH changes may have an impact on the trigeminal nerve and change trigeminal sensitivity and conductivity. Other studies have suggested that it may have an impact on the release of CGRP and thus on migraine pain.

Measuring pH in a nose during Bi-directional delivery with both the powder and liquid delivery devices resulted in unexpected results. Bi-directional blowing through both the powder and liquid devices without any release of substance caused a repeated and generally reproducible (sensor position may vary data) dip in pH by 0.1-0.2 pH units. This data is similar to what was observed with a 3 second burst of 15% and 45% carbon dioxide. In these studies the sensor was placed at the floor of the nose. Additional measurements with the sensor at the floor as well as the close to the roof of the nose were also conducted. In many instances, larger "dips" are observed when the sensor is placed towards the roof of the nose compared to the floor.

As hypothesized above, and based in part on previous measurements of NO, with the very low flow rates of carbon dioxide delivery, it takes time to achieve and increase carbon dioxide concentration in the upper part of the nose when carbon dioxide is delivered to the floor of the nose. Even with high concentrations of about 45% to about 100%, it may take more time that the 10 second pulses delivered to achieve the 6% which is achieved with Bi-Directional delivery. This could explain the "device effects" described above.

It is noteworthy that we are able to detect the "dips" in pH in direct response to Bi-Directional delivery. This data provides a scientific and logical explanation for the high placebo effects and some of the response rates. The very high response rate in moderate-to-severe migraine as early as 30 minutes in the above-described head-to-head trial, with 57% of the "blinded" attacks reduced from moderate/severe to mild or none at 30 minutes. This data was unexpected, regardless of the distribution between the two treatment groups. It is even more impressive to see that 95% of the attacks scored as severe were reduced to moderate, mild or none at 30 minutes.

Data described herein provides support to the hypothesis of device effects. Measurements with both the powder and liquid formulations result in similar data. Thus, it is the Bi-Directional methodology, rather that the specific device, that appears to have a significant effect It is noteworthy that carbon dioxide also has an effect in allergic rhinitis.

The nasal pH measurements were made using a Digitrapper pH 1.6 mm pH sensor and AccuView software. Digitrapper and software were provided by WinMed in Norway. In some embodiments, one or more probes can be located as shown generally in Figure 17. The probe may be located in either nasal passage.

Data showing pH as a function of exhalation flow, with a sensor probe located on same side towards nasal roof, using a powder device, is shown in Figure 18. Data showing pH as a function of exhalation flow using a liquid and a powder device are shown in Figure 19, with a pH sensor placed towards a roof of the nose approximately 4-5 cm from a nostril opening. Figure 20 illustrates data showing pH as a function of exhalation flow associated with a powder device, with a sensor located about 4 -5 cm into the nose at the floor and middle part of the nose. Fig. 21 shows additional data showing pH as a function of exhalation flow, again with a sensor located about 4-5 cm into the nose at the floor and middle part of the nose.

Shusterman (2003) delivered 3 second pulses of regular air (0%) and carbon dioxide at 15% and 45% to the nose. A pH sensor was placed along floor of the nose. Sampling frequency was 10 per second (10Hz). Data from this study is shown in Fig. 22. By way of comparison, the present data compared oral breathing, calm nasal breathing and calm nasal breathing before Breath Powered Bi-Directional delivery with powder and liquid devices. A sensor was located at about 4-5 cm into right nostril and the inhalation device inserted Into left nostril. Data associated with the method is shown in Figure 23.

In summary, the Breath Powered^{™} Bi-Directional^{™} delivery systems and methods offer, based on calculations, a higher amount of carbon dioxide per second delivery to the nose compared to 100% carbon dioxide delivered in trials showing conical effects in migraine and allergic rhinitis (Capnia - Casale 2008 & Spierings 2008). Breath Powered^{™} Bi-Directional^{™} delivery also shows similar reduction in pH levels in direct response to exhalations through the device as both 15% and 45% carbon dioxide are delivered in 3 second pulses 1 minute apart. These results suggest that the nature of Breath Powered^{™} Bi-Directional^{™} procedure can produce similar carbon dioxide exposures to the nasal mucosa as delivery of 100% used in trials has shown effects in migraine and perennial allergic rhinitis. These carbon dioxide effects of the Breath Powered^{™} Bi-Directional^{™} may be used in combination with positive pressure applied during the procedure, a high flow rate and changed flow pattern, improved airflow penetrating the nose, vibrating effect of the delivery device, removal of nitric oxide and increased exposure of carbon dioxide, which can cause effects on the trigeminal nerve and on mast cells.

### Example #8

A phase 2 trial with low-dose sumatriptan powder using a closed-palate Breath Powered^{™} device produced headache relief approaching levels previously reported with injections, but without triptan effects. Additional studies were undertaken to evaluate the efficacy and safety of this delivery regime as compared to placebo in patients with moderate-to-severe acute migraine headache. These studies included a phase 3, multicenter, randomized, double-blind, placebo-controlled, single-dose, parallel-group study, which was conducted in patients who had experienced between 1-8 migraines/month in the 12 months prior to screening. Each patient treated a single migraine headache of moderate or severe intensity with 2 doses (1 each nostril) of either a Breath Powered device containing 11 mg sumatriptan powder for a total dose of 22 mg or a matching device loaded with placebo (placebo device). The following efficacy outcomes were measured:
- Headache response (pain rated as mild or none) at 120 min (primary), and multiple time points up to 120 mins,
- Complete pain-free (freedom from headache pain) at multiple time points up to 120 mins,
- Time to meaningful relief (patient reported interpretation of headache pain response),
- Clinical disability and migraine-associated symptoms (photophobia, phonophobia, nausea and vomiting),
- Rescue medication use, and
- Sustained response/ sustained pain-free (headache response/complete pain-free at 120 min and no recurrence or use of rescue medication up to 24 and 48 h post-dose.

In total, 223 patients (mean age 42; 85% female) received treatment (112 sumatriptan powder; 111 placebo), Patient demographics and baseline characteristics are shown in Figure 24.

Headache response at 120 min (primary outcome) was 68% vs. 45% (P<.01). Headache response curves diverged early, reaching statistical significance at 30 min (42% vs. 27%; P<.05). In general, the present delivery regime was statistically superior to placebo for completed relief and sustained response and remained at 24 and 48 hours. Reductions were also seen in disability and migraine associated symptoms.

Results are shown in Figure 25. Generally, complete pain free (120 mins) was 37% vs. 17% (P<.01) and meaningful relief (120 mins) was 70% vs. 45% (P<.001). For the sustained response, at 24 hrs, it was 44% vs. 24% (P<.01) and at 48 hrs, it was 34% vs. 20% (P=.01). For sustained pain free, at 24 hrs, it was 28% vs. 12% (P=.005), and at 48 hrs, it was 20% vs. 9% (P=.02). In addition, reductions in nausea, phonophobia, and photophobia were reported in both groups (not significant vs. placebo). Significantly more patients using placebo (52%) than the present delivery regime (37%; P=.02) required rescue medication.

For the primary endpoint, 68% of patients using the present delivery regime reported headache relief at 120 min post-dose vs. 45% using placebo device (P<.01; Figure 26). Headache relief with the present delivery regime was achieved early, reaching statistical significance compared with placebo at 30 min (42% vs. 27%, P<,05; Figure 26). Consistent with results for the headache relief measure, significantly more patients using the present delivery regime experienced meaningful relief (Figure 27 - showing a proportion of patients with meaningful relief a following treatment with the present delivery regime or placebo device at 120 min post-dose (FAS)) and complete pain relief (Figure 28 - proportion of patients who achieved pain freedom at 120 min endpoint (FAS)) at the 120 min endpoint compared with placebo. More patients using the present delivery regime experienced sustained headache relief at 24 and 48 h vs. placebo device (Figure 26). More patients using the present delivery regime (28%) maintained pain freedom at 24 h without rescue medication vs. 12% using placebo (P<.01). Significantly fewer patients using the present delivery regime required rescue medication compared with placebo device (37% vs. 52%, P<.05). Clinical disability score was significantly improved in patients treated with the present delivery regime compared with placebo between 45 and 120 min inclusive (P<.05). The incidence of migraine-associated symptoms was substantially reduced at the 120 min endpoint (the present delivery regime vs. placebo device: nausea 19% vs. 21%, vomiting 2% vs. 0%, photophobia 48% vs. 60%, phonophobia 32% vs. 44%). These reductions did not reach significance between groups.

There were few systemic adverse effects (AEs) and none reported in more than one patient. AEs known as "triptan effects" are associated with formulations and doses that produce high plasma drug concentrations. There were also minimal triptan sensations. Specifically, there were no chest pressure/tightness, and only one patient reported mild, transient paraesthesias. The most common (>5%) AEs reported were product taste (22%), nasal discomfort (13%), and rhinitis (6%).

Unlike traditional nasal sprays, the present delivery regime uses a novel Breath Powered device to deliver powdered sumatriptan deep within nasal structures where it can be rapidly absorbed. This deep region is also extensively innervated by the trigeminal and olfactory nerves, theoretically offering potential for direct effects or nose-to-brain transport. The Breath Powered device delivers carbon dioxide locally and removes nitric oxide (NO). This effect may have contributed to both the placebo response seen in this study. The high placebo response may also be related to neurochemical effects of carbon dioxide delivery and/or removal of NO at the trigeminal nerve endings within the nasal cavity. NO is known to stimulate release of CGRP from the trigeminal neurons, a key mediator in the pathophysiology of migraine, whereas carbon dioxide inhibits CGRP release and may be beneficial in migraine modulation.

In conclusion, treatment with the present delivery regime produced fast and sustained migraine relief compared with placebo device with minimal triptan sensations. These data are consistent with results from an earlier phase 2 trial and suggest that the present delivery regime can offer an important therapeutic and practical option for acute migraine treatment.

### Example #9

In the examples and discussion provided above, carbon dioxide has been described as providing a mechanism to provide and/or enhance a therapeutic or pharmacokinetic effect and/or adjust the pH of a region within the nasal passage. Carbon dioxide may react within the nasal passage to lower pH. As described above, the concentration of delivered carbon dioxide can range from about 5 to about 6 % vol/vol. In other aspects, a therapeutic amount of carbon dioxide can include more than about 1% vol/vol carbon dioxide and less than about 10% vol/vol carbon dioxide.

A gas or fluid other than carbon dioxide could be used to provide pH adjustment, such as, for example, raising pH. It is also contemplated that one or more solid materials could be used to adjust pH within a nasal passage, with or without carbon dioxide or another gas or fluid. For example, fine particulate matter could be used to adjust the pH of an extracellular environment about tissue within the nasal passage.

In some embodiments, a pH adjusting material could include an acidic or a basic gas or buffer solution. The pH adjusting material could also form part of a formulation contained with or separate from a therapeutic agent. The pH adjusting material may adjust the pH by a known amount The known amount may be determined based on the requirements of an individual or group of individuals, a therapeutic agent, group of agents, or expected behavior of one or more agents. The known amount may range from about 0.01 to about 0.5 pH units, or about 0.1 to about 0.2 pH units.

Various mechanisms could be used to aerosolize or otherwise create an air flow containing the pH adjusting material. For example, a powder of pH adjusting material could be combined with the therapeutic agent in a capsule or blister pack. In another embodiment, one or more separate capsules or blister packs could be located adjacent to, upstream, or downstream of the therapeutic agent to provide pH adjustment prior to, simultaneously, or after the therapeutic agent is airborne. Mechanical, electrical, or chemical vibration mechanisms could also be used to release the pH adjusting material.

### Example #10

In a 3-month placebo controlled study in 109 patients with chronic rhinosinusitis (CRS) with nasal polyps, delivery of fluticasone (400 µg b.i.d.) with a Breath Powered^{™} liquid drug delivery device was reported to be well tolerated and to produce a large magnitude of reduction in both symptoms and the overall polyp score.

Particularly notable relative to expectations with standard nasal spray delivery, complete elimination of the polyps in close to 20 % of the subjects was reported after 3 months. The proportion of subjects with improvement in summed polyp score was significantly higher with the present delivery regime as compared with placebo at 4, 8, and 12 weeks (22 % vs. 7 %, p=0.01 1, 43 % vs. 7 %, p<0.001, 57 % vs. 9 %, p<0.001). Despite relatively lower baseline polyp scores after 12 weeks, the summed polyp score was significantly reduced from 2.8 to 1.8 in the active treatment group, whereas a minor increase in polyp score was seen in the placebo group (-0.98 vs. +0.23, p<0.001).

Peak nasal inspiratory flow (PNIF) increased progressively during treatment with the present delivery regime (p<0.001). Combined symptom score, nasal blockage, discomfort, rhinitis symptoms, and sense of smell were all significantly improved.

The highly significant progressive treatment effect of the present delivery regime was observed regardless of baseline polyp score. Previous sinus surgery had no Impact on the efficacy. Coupled with the complete removal of polyps in many patients with small polyps, this suggests that improved deposition to target sites achieved with the Breath Powered^{™} delivery device may translate into true clinical benefits and possibly reduced need for surgery.

### Example #11

Using the same drug-device combination product as Example #10. a small placebo controlled study (N=20) was performed in patients with post-surgical recalcitrant CRS without polyps, producing clinically significant improvements on both objective measures and subjective symptoms.

Endoscopy score for edema showed a significant and progressive improvement [12 weeks (median scores): the present delivery regime -4.0, vs. placebo - 1.0, p=0.015].

Peak nasal inspiratory flow (PNIF) increased significantly during treatment with the present delivery regime as compared to placebo (4weeks: p=0.006; 8 weeks: p=0.03). After 12 weeks, MRI scores in the group receiving the present delivery regime improved against baseline (p=0.039), and a non-significant trend was seen vs. placebo.

The nasal RSOM-31 subscale was also significantly improved with treatment using the present delivery regime (4 weeks: p=0.009, 8 weeks: p=0.016, 12 weeks: NS). Sense of smell, nasal discomfort, and combined score were all significantly improved (p<0.05). Notably, this is a condition marked by many recent negative placebo-controlled trials. This context, in addition to comparison with historical data in similar patient populations, again suggests that breath-powered bi-directional delivery is capable of producing superior deep nasal deposition in clinical practice (improved targeting of the middle meatus in this case) which can translate into improved clinical response.

In relation to the administration of fluticasone, the present inventors have established that it is possible to deliver a significantly greater dose than conventionally, 400 ug or 800 ug twice daily as compared to 200 ug daily, and yet result in no significantly greater bioavailability.

As described above, the present disclosure provides a method of treating a patient. The treatment can include one or more steps, wherein a first step can include administering a therapeutic agent. A second step can include delivering carbon dioxide or a pH adjusting material to one or more regions of the nasal passage, as described above. The order of the steps can be interchanged, so the second step occurs before the first. It is also contemplated that both steps, or more, may occur simultaneously.

As discussed above, it is postulated that the effect of carbon dioxide, particularly in terms of pH and the NO concentration, and increased pressure produced by the device within the nasal cavity on the trigeminal nerve and sphenopalatine ganglion results in a higher overall response rate, especially in the oral tablet group at early time-points.

Finally, it will be understood that the present disclosure has been described in various embodiments and can be modified in many different ways without departing from the scope of the disclosure as defined by the appended claims. For example, the present disclosure has been exemplified in relation to sumatriptan, but it will be understood that the present disclosure has application to many other substances, including other triptans, such as risatriptan, naratriptan, eletriptan, frovatriptan and zolmitriptan, and other analgesics, such as ergotamines, including dihydroergotamine mesylate, ergonovine maleate and ergotamine tartarate with caffeine, fentanyl, oxycondone, hydromorphone, morphine, codeine, ketobbemidone, cocaine and opiods in general. The present disclosure also has application to benzodiazepines, such as midazolam. The present disclosure further has application In relation to non-steroidal antiinflammatory drugs (NSAIDs), for example, aspirin, ibuprofen, naproxen, indomethacin, diclofenac and ketoprofen.

The present disclosure still further has application in relation to proteins and peptides, in particular having a molecular weight greater than 1000 g/mol, which typically have a very low oral bio-availability, often less than 1%. Particular examples include insulin, including its analogues and derivatives, desmopressin and calcitonin. The present disclosure yet still further has application in relation to powder vaccines, immunomodulators and immunostimulators. In summary, the present disclosure has application in relation to the following broad definitions of molecules.

Small molecules (<1000) with relatively fast nasal absorption and high nasal BA, such as fentanyl, midazolam and oxycodone. The present disclosure suggests far more rapid CNS effects than compared to the prior art nasal administration systems, which could be because of differences between arterial and venous concentrations, where arterial absorption is between about 25% and 50% greater than venous absorption, possible "counter current" transport to the sinus cavernous and the carotid artery, which must pass the BBB, which has been shown to be about 25% greater in animal studies, and possible direct N2B transport along the olfactory and trigeminal nerves (Einer-Jensen, N et al, Pharmacol. Toxicol., 87(6), 2000, pages 276 to 278, Einer-Jensen, N et al, Exp. Brain Res., 130(2), 2000, pages 216 to 220, and Dale, O et al, intranasal Midazolam: a comparison of two delivery devices in human volunteers, J. Pharmacy and Pharmacology, 58, 2006, pages 1311 to 1318). N2B transport and clinical effects via the trigeminal nerves are not, however, necessarily reflected in the traditional PK profile.

Small and medium sized molecules with relatively poor BA, such as sumatriptan and zolmitriptan. For the sumatriptan powder of the present disclosure, sumatriptan passes the BBB relatively poorly, but animal studies suggest that sumatriptan can be transported directly to the brain by direct N2B mechanisms (Gladstone, J P, Newer formulations of triptans: Advances in migraine treatment, Drugs, 63, 2003, pages 2285 to 2305). The present disclosure provides for Increased absorption, which is particularly relevant where rapid absorption and a fast onset of action are desirable. The present disclosure suggests more rapid CNS effects, which could be because of possible direct N2B uptake, possible "counter current" transport to the sinus cavernous and the carotid artery, where the molecule is able to pass the BBB, and possible direct N2B transport along the olfactory and trigeminal nerves.

Larger molecules (>1000), Including peptides and proteins, which have low nasal BA, typically between about 3 and 15%, and very poor oral BA, typically less than 1%, because of degradation in the GI tract. The present disclosure, in providing a powder formulation, is particularly suited to the delivery of peptides and proteins, where the powder can provide for improved nasal absorption, but also can have improved stability. For these substances, it is postulated that there may be a dedicated transport mechanism along the olfactory and trigeminal nerves directly to the cerebral structures, which is not via the CSF. As such, measurements from the CSF may not show the presence of active substance, but a substantial effect may be present in the brain and exert clinical effects, as exemplified in a recent study (Thorne, R G et al, Delivery of insulin-like growth factor-I to the rat brain and spinal cord along olfactory and trigeminal pathways following intranasal administration, Neuroscience, 127(2), 2004, pages 481 to 496).

While principles of the present disclosure are described herein with reference to illustrative embodiments for particular applications, it should be understood that the disclosure is not limited thereto. Those having ordinary skill in the art and access to the teachings provided herein will recognize additional modifications, applications, embodiments, and substitution of equivalents all fall within the scope of the embodiments described herein. Accordingly, the disclosure is not to be considered as limited by the foregoing description.

### STATEMENTS

Furthermore, the present invention is represented by the following statements of invention.
1. An apparatus or method for therapeutically treating a patient, comprising:
   administering, in a first step, a therapeutic agent; and
   delivering, in a second step, to a location at an interior of a nasal passage of the patient a therapeutic amount of at least one of carbon dioxide or a pH adjusting material, wherein the first step is performed before, after, before and after and/or at the same time as the second step.
2. The apparatus or method of statement 1, wherein the first step Is performed before the second step.
3. The apparatus or method of statement 1, wherein the first step is performed after the second step.
4. The apparatus or method of statement 1, wherein the second step is performed both before and after the first step and/or the first step is performed at the same time as the second step.
5. The apparatus or method of statement 1, wherein the location includes an upper posterior region of the nasal passage.
6. The apparatus or method of statement 1, wherein the therapeutic agent includes sumatriptan, optionally in the form of sumatriptan succinate, optionally administered as a powder aerosol.
7. The apparatus or method of statement 1, wherein the therapeutic agent includes a topical steroid, optionally fluticasone, optionally in the form of fluticasone propionate, optionally administered as a liquid aerosol.
8. The apparatus or method of statement 7, wherein the therapeutic agent is delivered in amount of at least 100 ug, optionally twice daily.
9. The apparatus or method of statement 8, wherein the therapeutic agent is delivered in amount of at least 200 ug, optionally twice daily.
10. The apparatus or method of statement 9, wherein the therapeutic agent Is delivered in amount of at least 400 ug, optionally twice daily.
11. The apparatus or method of statement 8, wherein the delivering comprises:
   placing a mouthpiece into a mouth of the patient and a nosepiece into a nostril of the patient, the mouthpiece being fluidly connected to the nosepiece; and
   the patient exhaling a breath into the mouthpiece to create a fluid flow out of the nosepiece;
   and wherein the therapeutic agent when intranasally administered has a systemic bioavailability or pharmacokinetic (PK) profile which is equivalent to or not greater than the systemic bioavailability of 50 ug of the therapeutic agent when intranasally administered without the delivering.
12. The apparatus or method of statement 1, wherein the second step adjusts a pH in the nasal passage location by an amount ranging from about 0.01 to about 0.5 pH units.
13. The apparatus or method of statement 12, wherein the amount ranges from about 0.1 to about 0.2 pH units.
14. The apparatus or method of statement 1, wherein the second step comprises delivering a concentration of carbon dioxide from about 1% vol/vol to about 10% vol/vol carbon dioxide.
15. The apparatus or method of statement 14, wherein the concentration of carbon dioxide is from about 5% to about 6% vol/vol carbon dioxide.
16. The apparatus or method of statement 1, wherein delivering comprises:
   placing a mouthpiece Into a mouth of the patient and a nosepiece into a nostril of the patient;
   the patient exhaling a breath into the mouthpiece to create a fluid flow out of the nosepiece; and
   directing the fluid flow to the location within the nasal passage.
17. The method of statement 16, wherein delivering further
   comprises: adjusting a pH at the location by controlling the fluid flow.
18. The apparatus or method of statement 17, wherein controlling the fluid flow includes at least one of controlling a duration, a rate, a pressure, and a composition of the fluid flow.
19. The apparatus or method of statement 18, comprising controlling the fluid flow duration to be in a range of from about 2 to about 3 seconds.
20. The apparatus or method of statement 19, comprising controlling the fluid flow rate at at least 10 L/min, optionally at least 20 L/min, and optionally at about 30 L/min.
21. The apparatus or method of statement 16, wherein the delivering further comprises:
   placing the nosepiece into a second nostril of the patient;
   the patient exhaling a breath into the mouthpiece to create a second fluid flow out of the nosepiece; and
   directing the second fluid flow to a second location within a second nasal passage.
22. An apparatus or method for increasing a therapeutic effect of a pharmaceutical agent delivered to a patient, comprising:
   delivering a fluid flow to a nasal passage of the patient to deliver about 5% to about 6% vol/vol carbon dioxide to a posterior region of the nasal passage; and
   administering a dose of the pharmaceutical agent to the patient.
23. The apparatus or method of statement 22, further including lowering a pH of the posterior region of the nasal passage.
24. The apparatus or method of statement 22, wherein the pharmaceutical agent includes sumatriptan, optionally in the form of sumatriptan succinate, optionally administered as a powder aerosol.
25. The apparatus or method of statement 22, wherein the pharmaceutical agent includes a topical steroid, optionally fluticasone, optionally in the form of fluticasone propionate, optionally administered as a liquid aerosol.
26. The apparatus or method of statement 25, wherein the pharmaceutical agent Is delivered In amount of at least 100 ug, optionally twice dally.
27. The apparatus or method of statement 26, wherein the pharmaceutical agent Is delivered In amount of at least 200 ug, optionally twice daily.
28. The apparatus or method of statement 27, wherein the pharmaceutical agent Is delivered in amount of at least 400 ug, optionally twice daily.
29. The apparatus or method of statement 26, wherein the delivering comprises:
   placing a mouthpiece into a mouth of the patient and a nosepiece into a nostril of the patient, the mouthpiece being fluidly connected to the nosepiece; and
   the patient exhaling a breath into the mouthpiece to create a fluid flow out of the nosepiece;
   and wherein the pharmaceutical agent when intranasally administered has a systemic bioavailability or pharmacokinetic (PK) profile which is equivalent to or not greater than the systemic bioavailability or pharmacokinetic (PK) profile of 50 ug of the pharmaceutical agent when intranasally administered without the delivering.
30. An apparatus or method for treating a patient, comprising delivering a concentration of about 5% to about 6% vol/vol carbon dioxide to a nostril of the patient to lower a pH of an upper posterior region of the nasal passage by at least about 0.1 pH units to provide a therapeutic or pharmacokinetic effect
31. The apparatus or method of statement 30, further including administering a therapeutic agent to the patient at least one of orally, intranasally, intravenously, and sub-cutaneously.
32. The apparatus or method of statement 30, further Including administering a dose of sumatriptan powder intranasally.
33. The apparatus or method of statement 32, the low dose includes less than 20 mg of a sumatriptan powder, optionally about 16 mg of sumatriptan powder, optionally in the form of sumatriptan succinate.
34. The apparatus or method of statement 30, further including administering a topical steroid, optionally fluticasone, optionally in the form of fluticasone propionate, optionally administered as a liquid aerosol.
35. The apparatus or method of statement 34, wherein the steroid is delivered in amount of at least 100 ug, optionally twice daily.
36. The apparatus or method of statement 35, wherein the steroid is delivered in amount of at least 200 ug, optionally twice daily.
37. The apparatus or method of statement 36, wherein the steroid is delivered in amount of at least 400 ug, optionally twice daily.
38. The apparatus or method of statement 30, wherein the delivering comprises:
   placing a mouthpiece into a mouth of the patient and a nosepiece into a nostril of the patient, the mouthpiece being fluidly connected to the nosepiece; and
   the patient exhaling a breath into the mouthpiece to create a fluid flow out of the nosepiece;
   and wherein the steroid when intranasally administered has a systemic bioavailability or pharmacokinetic (PK) profile which is equivalent to or not greater than the systemic bioavailability or pharmacokinetic (PK) profile of 50 ug of the steroid when intranasally administered without the delivering.
39. The apparatus or method of statement 30, wherein the therapeutic or
   pharmacokinetic effect treats at least one of migraine and allergic rhinitis.
40. The apparatus or method of statement 30, further including substantially closing a soft palate of the patient during the delivery step.

## Claims

1. A therapeutic agent including fluticasone for treating chronic rhinosinusitis with or without nasal polyps in a patient and carbon dioxide as a pH reducing material in combination, **characterized in that** the fluticasone is delivered in amount of 100 µg to 400 µg twice daily and administration is by a delivery device comprising a mouthpiece and a nosepiece, by placing the mouthpiece into a mouth of the patient and the nosepiece into a nostril of the patient and the patient exhaling a breath into the mouthpiece to create a fluid flow out of the nosepiece into the nostril and deliver the fluticasone and the carbon dioxide from the nosepiece to a location within a nasal passage of the patient, wherein a pH in the nasal passage location is reduced by an amount ranging from about 0.1 pH units to about 0.2 pH units by controlling the fluid flow to deliver a concentration of the carbon dioxide of from about 5% vol/vol to about 6% vol/vol carbon dioxide at a fluid flow rate of at least 20 L/min for a duration of from about 2 seconds to about 3 seconds and the carbon dioxide is delivered before or at the same time as the fluticasone.

2. The therapeutic agent of claim 1, wherein the nasal passage location includes an upper posterior region of the nasal passage.

3. The therapeutic agent of claim 1, wherein the fluticasone is in the form of fluticasone propionate.

4. The therapeutic agent of claim 3, wherein the fluticasone is administered as a liquid aerosol.

5. The therapeutic agent of claim 1, wherein the fluticasone is delivered in amount of 100 µg twice daily.

6. The therapeutic agent of claim 1, wherein the fluticasone is delivered in amount of 200 µg twice daily.

7. The therapeutic agent of claim 1, wherein the fluticasone is delivered in amount of 400 µg twice daily.

8. An apparatus or method for therapeutically treating a patient, comprising:
administering, in a first step, a therapeutic agent; and
delivering, in a second step, to a location at an interior of a nasal passage of the patient a therapeutic amount of at least one of carbon dioxide or a pH adjusting material, wherein the first step is performed before, after, before and after and/or at the same time as the second step.

9. An apparatus or method for increasing a therapeutic effect of a pharmaceutical agent delivered to a patient, comprising:
delivering a fluid flow to a nasal passage of the patient to deliver about 5% to about 6% vol/vol carbon dioxide to a posterior region of the nasal passage; and
administering a dose of the pharmaceutical agent to the patient.

10. An apparatus or method for treating a patient, comprising:
delivering a concentration of about 5% to about 6% vol/vol carbon dioxide to a nostril of the patient to lower a pH of an upper posterior region of the nasal passage by at least about 0.1 pH units to provide a therapeutic or pharmacokinetic effect.
